(19) Europäisches Patentamt
European Patent Office
Office européen des brevets

(11) EP 2 062 882 B1

(12) **EUROPEAN PATENT SPECIFICATION**

(45) Date of publication and mention of the grant of the patent:
**28.09.2011 Bulletin 2011/39**

(21) Application number: **07850471.9**

(22) Date of filing: **12.12.2007**

(51) Int Cl.:
***C07D 307/58*** *(2006.01)* ***C08F 24/00*** *(2006.01)*
***C08F 220/36*** *(2006.01)* ***C09K 19/38*** *(2006.01)*
***G02B 5/30*** *(2006.01)* ***G02F 1/1335*** *(2006.01)*
***G02F 1/13363*** *(2006.01)*

(86) International application number:
**PCT/JP2007/073920**

(87) International publication number:
**WO 2008/072652 (19.06.2008 Gazette 2008/25)**

(54) **POLYMERIZABLE LIQUID CRYSTAL COMPOUND, POLYMERIZABLE LIQUID CRYSTAL COMPOSITION, AND ALIGNMENT FILM**

FLÜSSIGKRISTALLAUSRICHTUNGSFOLIENZUSAMMENSETZUNG, POLYMERISIERBARE FLÜSSIGKRISTALLZUSAMMENSETZUNG UND AUSRICHTUNGSFOLIE DAFÜR

COMPOSE CRISTAL LIQUIDE POLYMERISABLE, COMPOSITION DE CRISTAUX LIQUIDES POLYMERISABLES ET FILM D'ALIGNEMENT

(84) Designated Contracting States:
**DE NL**

(30) Priority: **15.12.2006 JP 2006338201**

(43) Date of publication of application:
**27.05.2009 Bulletin 2009/22**

(73) Proprietor: **Nissan Chemical Industries, Ltd.**
**Chiyoda-ku,**
**Tokyo 101-0054 (JP)**

(72) Inventor: **SAHADE, Daniel Antonio**
**Funabashi-shi**
**Chiba 274-0052 (JP)**

(74) Representative: **Stoner, Gerard Patrick et al**
**Mewburn Ellis LLP**
**33 Gutter Lane**
**London**
**EC2V 8AS (GB)**

(56) References cited:
**WO-A1-2006/115112 JP-A- 09 241 249**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 062 882 B1

Printed by Jouve, 75001 PARIS (FR)

## Description

### TECHNICAL FIELD

The present invention relates to a polymerizable liquid crystal compound having polymerizability and liquid crystallinity, and a composition including the same and a polymer obtained by use of them and more particularly, to a polymerizable liquid crystal compound that is conveniently usable, for example, for display devices and as a material having optical characteristics such as a recording material, especially, as an optical compensation film such as a polarizer plate, phase difference plate or the like for liquid crystal displays, and a composition including the same and a polymer obtained by use of them.

### BACKGROUND ART

In view of requirements for an improvement in display quality of liquid crystal display devices and weight saving, there has been a demand of polymer films whose internal molecule alignment structure is controlled for use as an optical compensation film such as a polarizer plate, a phase difference plate or the like. In order to meet the above requirement, there have been developed films that make use of optical anisotropy of polymerizable liquid crystal compounds.
The polymerizable liquid crystal compounds used herein are usually those liquid crystal compounds having a polymerizable group and liquid crystal structure moieties (structural moieties including a spacer moiety and a mesogenic moiety), and an acryl group is in wide use as such a polymerizable group.
In general, such a polymerizable liquid crystal compound is converted to a polymer (film) according to a method of polymerization by irradiation of a radiation ray such as W light or the like.
For instance, there are known methods including, for example, a method wherein a specific type of polymerizable liquid crystal compound having an acryl group is disposed between supports, under which while keeping the compound in a liquid crystal condition, a radiation ray is irradiated thereat to obtain a polymer (see Patent Document 1) and a method wherein a mixture of two types of acryl group-bearing polymerizable liquid crystal compounds having an acryl group or a composition obtained by mixing a chiral liquid crystal with the above mixture is admixed with a photopolymerization initiator, followed by irradiation of UV light thereon to obtain a polymer (see Patent Document 2).
The polymers (films) obtained by the above methods are mounted not only in display devices such as a monitor or a television set for used as a film for polarizer plate, phase difference plate or the like, but also in display devices employed in a high-temperature environment such as in automotives. Accordingly, to keep transparency in the high-temperature environment is very important for use as a display device material.
However, where the film obtained from a polymerizable liquid crystal compound has a glass transition temperature (hereinafter abbreviated as Tg) that is lower than a temperature in a use environment or is placed especially in a high-temperature environment, its alignment is disturbed because of the microscopic fluctuation of the molecules, with the possibility that the optical anisotropy may lower considerably.
Further, studies on a simplification of the process using these materials as an In Cell phase difference film have recently been in positive progress in the field of displays. It is required that the materials used in this In Cell technique have higher thermal stability and chemical resistance.
Patent Document 1: JP-A 62-70407
Patent Document 2: JP-A 9-208957
JP-A 9-241249 discloses polymerizable optically active compounds and liquid crystalline compositions. WO-2006/115112 also discloses polymerizable liquid crystalline compositions for optical applications.

### DISCLOSURE OF THE INVENTION

### PROBLEMS TO BE SOLVED BY THE INVENTION

The present invention has been made under these circumstances and has for its object the provision of a polymerizable liquid crystal compound capable of yielding a polymer that has excellent optical anisotropy, is able to keep a stable retardation value and transparency at high temperatures, and is excellent in chemical and heat resistances, and a polymerizable liquid crystal composition including the same and polymers thereof.

### MEANS FOR SOLVING THE PROBLEMS

The present inventors made intensive studies in order to solve the above problem and, as a result, found that

a given type of polymerizable liquid crystal compound having an α-methylene-γ-butyrolactone moiety has liquid crystallinity, is excellent in polymerizability of itself and is able to yield a stable liquid crystalline composition and that the polymer or film obtained from the liquid crystalline composition has an excellent heat resistance with respect to optical anisotropy and transparency. Based on these findings, the present invention has been attained.

More particularly, the present invention provides:

1. A polymerizable liquid crystal compound, characterized by being represented by the following formula [1]

[Chemical Formula 1]

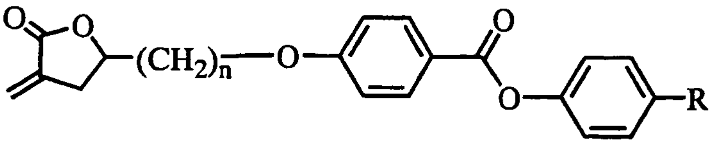

[1]

(wherein R represents an organic group represented by the following formula [A-1], [B-1], [B-2] or [C-1], and n is an integer of 2 to 9),

[Chemical Formula 2]

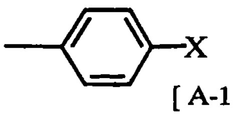

[A-1]

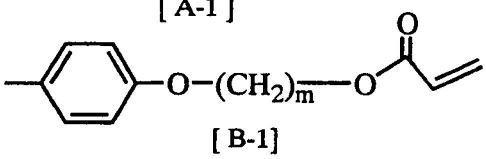

[B-1]

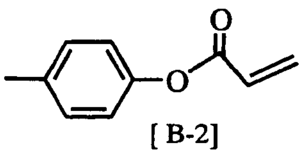

[B-2]

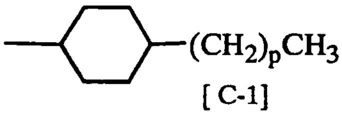

[C-1]

(wherein X represents a hydrogen atom, a halogen atom, a cyano group or an alkoxy group, m is an integer of 2 to 10, and p is an integer of 0 to 6);

2. The polymerizable liquid crystal compound of 1 wherein R is an organic group represented by the above formula [B-1] or [B-2];

3. A polymerizable liquid crystal composition including at least one of the polymerizable liquid crystal compound of 1 or 2;

4. A coating solution for forming an alignment film including at least one of the polymerizable liquid crystal compound of 1 or 2;

5. An alignment film obtained from the polymerizable liquid crystal composition of 3;

6. An alignment film obtained from the coating solution for forming an alignment film of 4;

7. An optical film provided with the alignment film of 5 or 6;

8. A display device provided with the alignment film of 5 or 6; and

9. A compound represented by the formula [2]

[Chemical Formula 3]

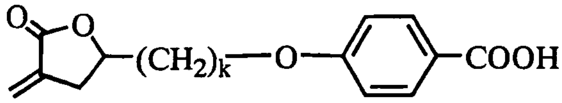

[2]

(wherein k is an integer of 2 to 9).

EFFECT OF THE INVENTION

The polymerizable liquid crystal compound of the present invention and the composition including the same can yield a polymer that has not only excellent optical anisotropy, but also exhibits a high chemical resistance with respect to the optical anisotropy, and is stable relative to the anisotropy and transparent in a high-temperature environment.
Accordingly, the polymer obtained from the composition including the polymerizable liquid crystal compound is utilizable as an optical anisotropic film such as a polarizer plate or a phase difference plate or the like and is much suited for use in a high-temperature environment.

BRIEF DESCRIPTION OF THE DRAWINGS

Fig. 1 is a graph showing an incident angle dependence of a retardation value of a film of Example 18 in an unbaked condition (A), thermal stability based on the incident angle dependence of a retardation value after baking of 200°C/1 hour (B) and thermal stability based on the incident angle dependence of a retardation value after baking 230°C/1 hour (C).
Fig. 2 is a graph showing an incident angle dependence of a retardation value of a film of Comparative Example 5 in an unbaked condition (A), and thermal stability based on the incident angle dependence of a retardation value after baking of 200°C/1 hour (B).

BEST MODE FOR CARRYING OUT THE INVENTION

Meanings of some terms used herein are those set out below.
"Polymerizable liquid crystal compound" means a compound that has a polymerizable moiety such as an acryl group, an $\alpha$-methylene lactone ring or the like and a liquid crystal structural moiety in the molecule and shows a liquid crystal phase. This "liquid crystal structure" means a structure having a spacer moiety and a mesogenic moiety, which are generally used in case of expressing liquid crystal molecules. "Liquid crystal composition" means a composition that has a characteristic of showing a liquid crystal phase. "Liquid crystallinity" means showing a liquid crystal phase.
The present invention is now described in more detail.

[Polymerizable liquid crystal compound]

The polymerizable liquid crystal compound of the present invention is represented by the following formula [1].
[Chemical Formula 4]

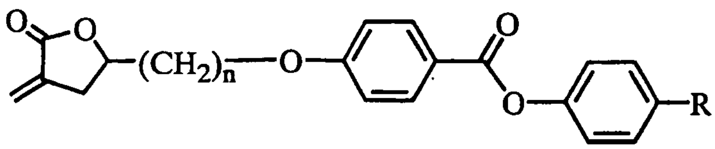

[1]

(In the formula, R represents an organic group represented by the following formula [A-1], [B-1], [B-2] or [C-1] and n is an integer of 2 to 9.)

[Chemical Formula 5]

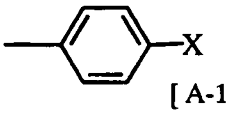

[A-1]

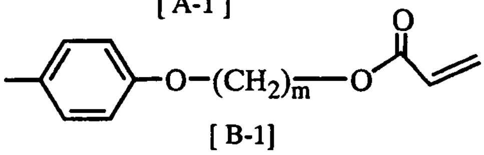

[B-1]

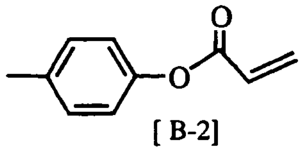

[B-2]

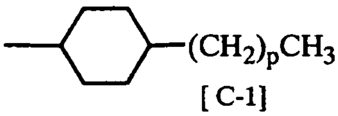

[C-1]

(In the formula, X represents a hydrogen atom, a halogen atom, a cyano group or an alkoxy group, m is an integer of 2 to 10, and p is an integer of 0 to 6.)

The compound represented by the formula [1] is one that has a lactone ring and a liquid crystal structural moiety and is a polymerizable liquid crystal compound having an α-methylene-γ-butyrolactone moiety.

The α-methylene-γ-butyrolactone is less influenced among α-alkylidene-γ-butyrolactones having a polymerizable group by the steric hindrance and can show such an excellent effect of high polymerizability. The compound is effective in imparting high Tg and heat resistance to the polymer obtained by use thereof.

In the formula [1], the repeating moiety of the methylene group is one that is a so-called spacer moiety. In the formula, n represents the number of repetitions of the methylene group and is an integer of 2 to 9, preferably 4 to 6.

In the formula [1], R is an organic group represented by the above formula [A-1], [B-1], [B-2] or [C-1].

In the formula [A-1], X is a hydrogen atom, a halogen atom (fluorine atom, chlorine atom, bromine atom or iodine atom), a cyano group or an alkoxy group, of which a hydrogen atom, a halogen atom, a cyano group or an alkoxy group having 1 to 9 carbon atoms is preferred and a hydrogen atom, a cyano group or an alkoxy group having 1 to 6 carbon atoms is more preferred.

In the formula [B-1], m is an integer of 2 to 10, more preferably 2 to 6.

In the formula [C-1], p is an integer of 0 to 9, preferably 2 to 6.

Of these, the organic group represented by the formula [B-1] or [B-2] is preferred and the organic group represented by the formula [B-2] is more preferred from the standpoint that the compound can be improved in thermal stability.

Especially, the polymerizable liquid crystal compound of the formula [1] wherein n is an integer of 4 to 6 and R is a group of the [B-2] is suited in view of the fact that the polymer obtained from a composition including such a compound has high thermal stability.

The polymerizable liquid crystal compound represented by the above formula [1] exhibits a liquid crystal phase such as a smectic phase or a nematic phase. This characteristic is useful in the field of applications making use of optical anisotropy such as of a polarizer plate, a phase difference plate or the like.

Examples of the above polymerizable liquid crystal compound include those compounds (1) to (78) indicated below although not limited thereto.

[Chemical Formula 6]

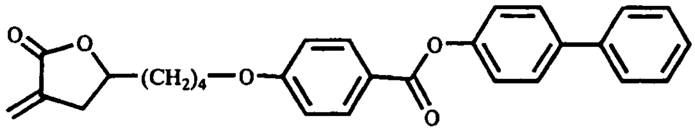

(1)

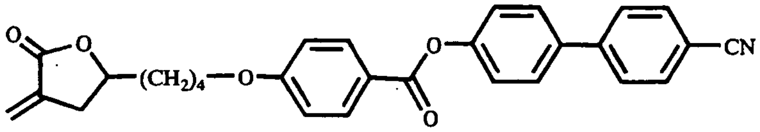

(2)

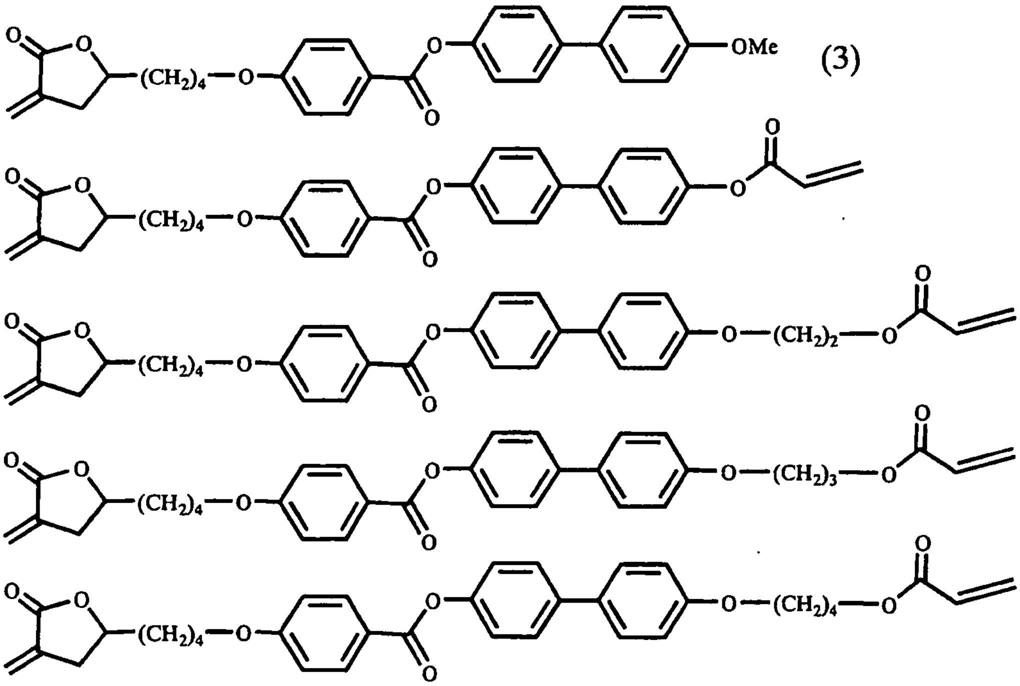

(3)

(4)

(5)

(6)

(7)

[Chemical Formula 7]

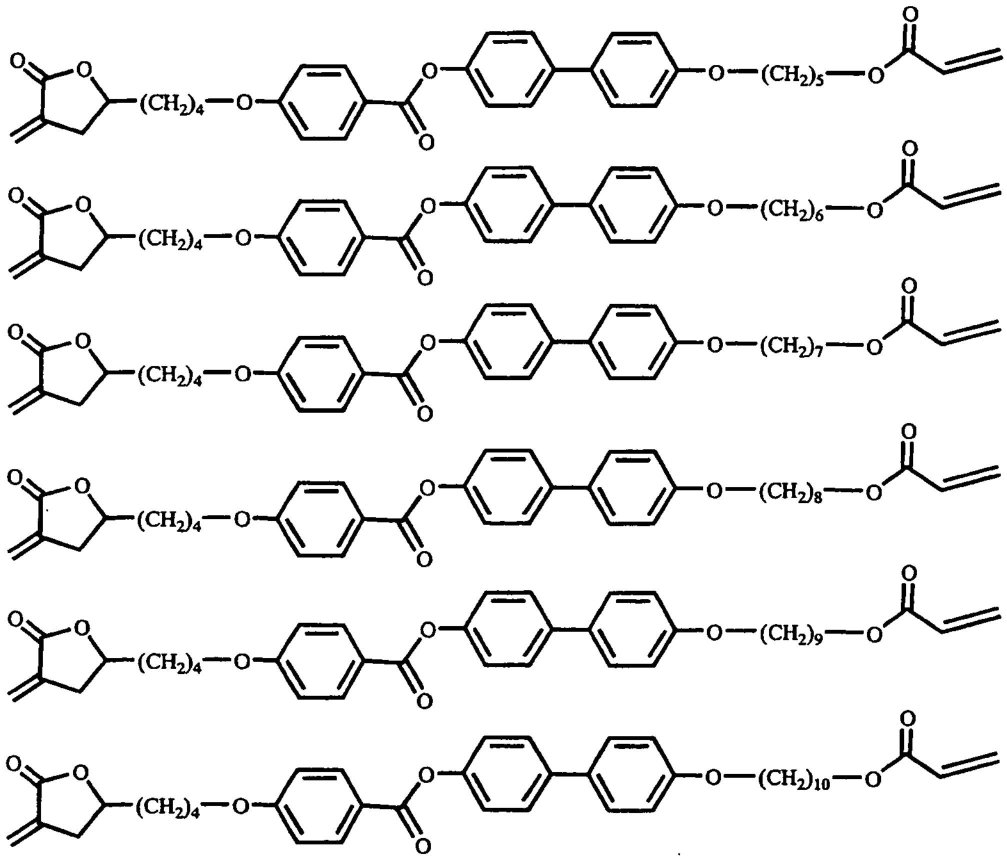

(8)

(9)

(10)

(11)

(12)

(13)

[Chemical Formula 8]

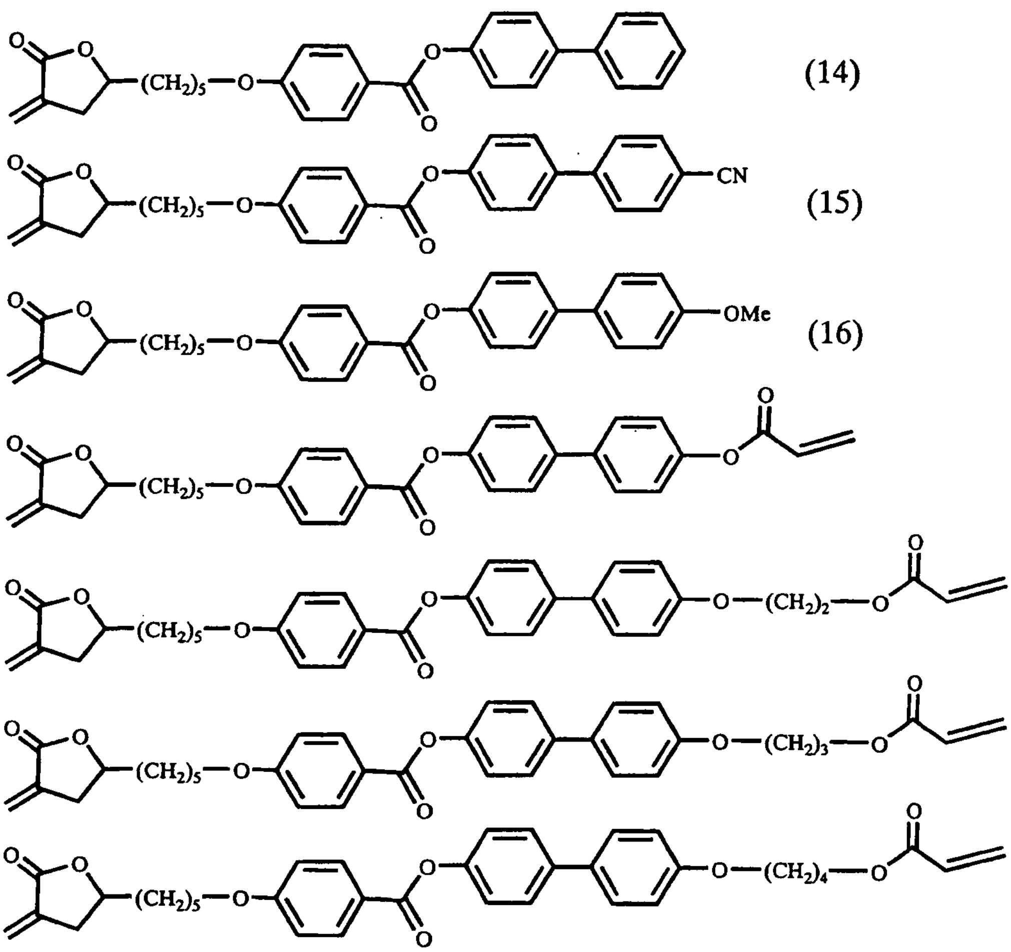

(14)

(15)

(16)

(17)

(18)

(19)

(20)

[Chemical Formula 9]

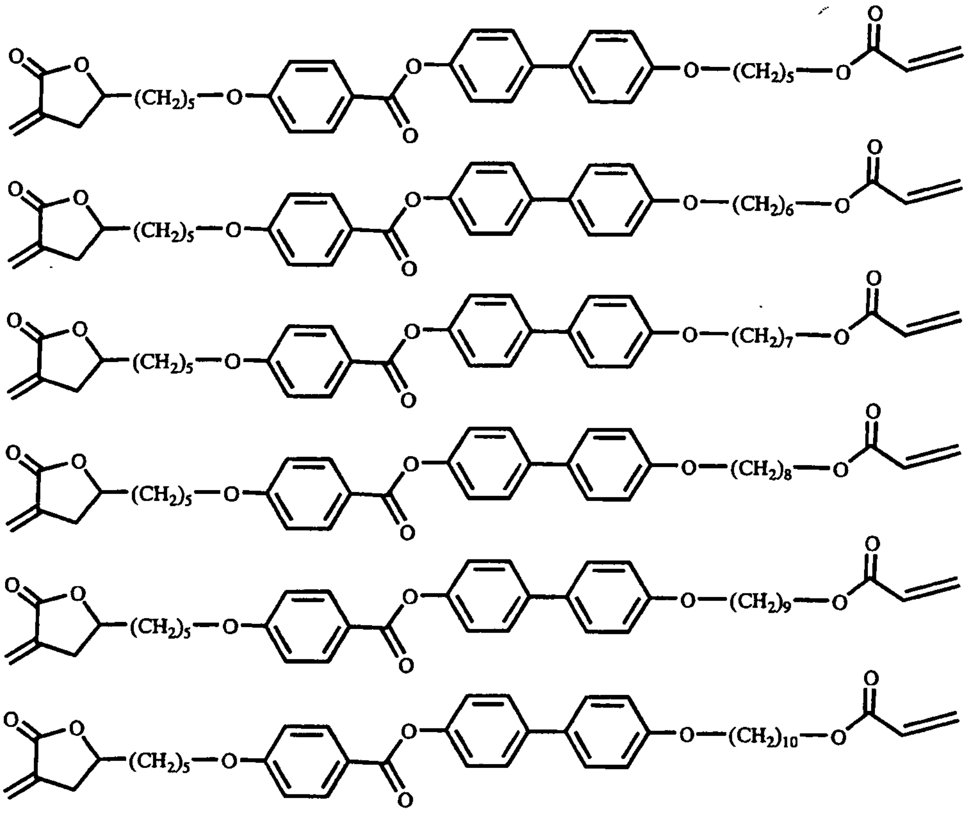

(21)

(22)

(23)

(24)

(25)

(26)

[Chemical Formula 10]

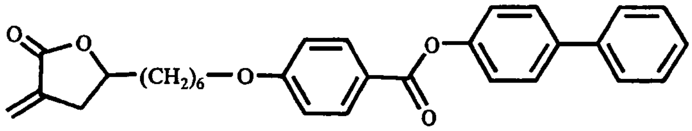
(27)

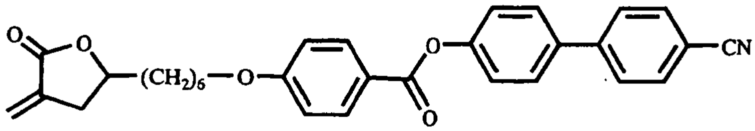
(28)

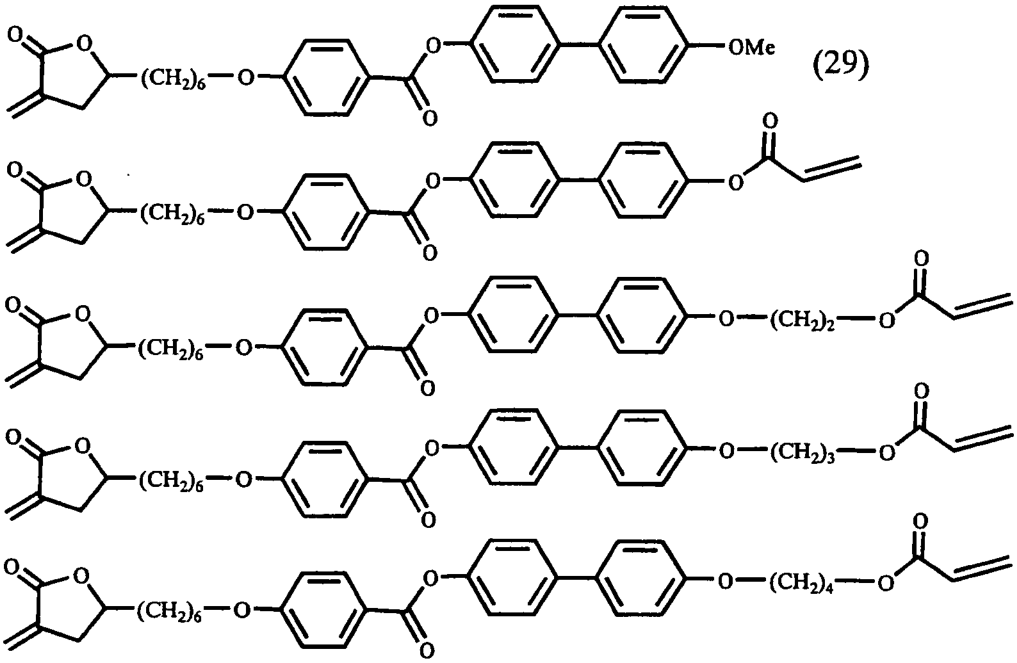
(29)

(30)

(31)

(32)

(33)

[Chemical Formula 11]

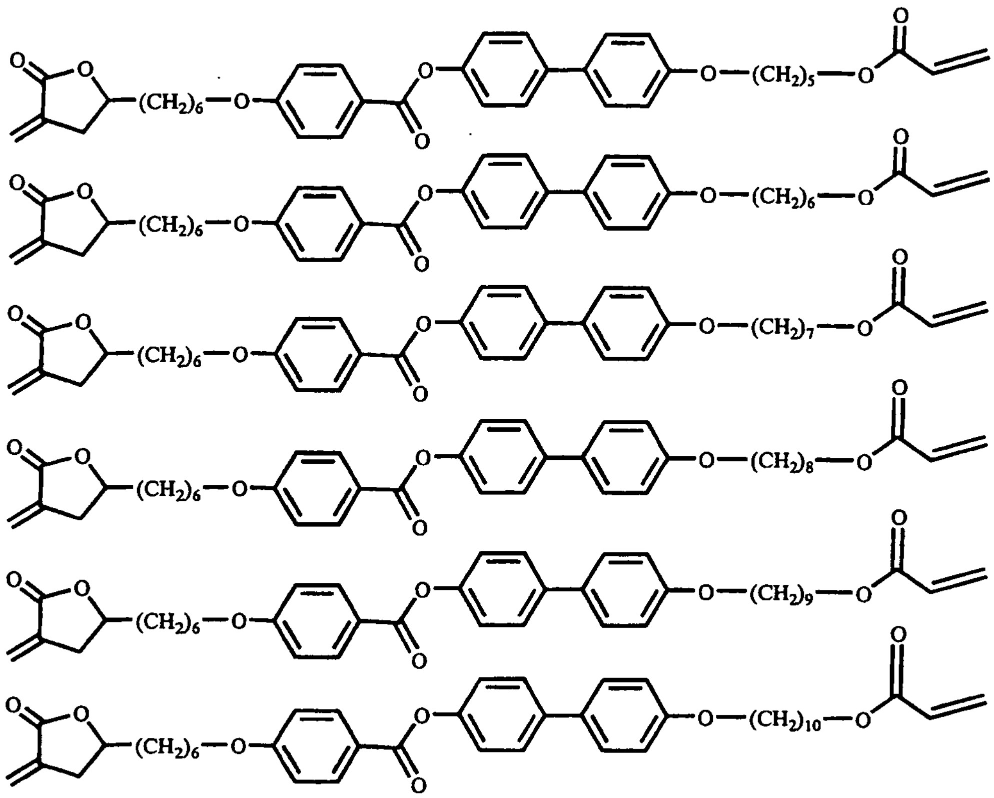

(34)

(35)

(36)

(37)

(38)

(39)

[Chemical Formula 12]

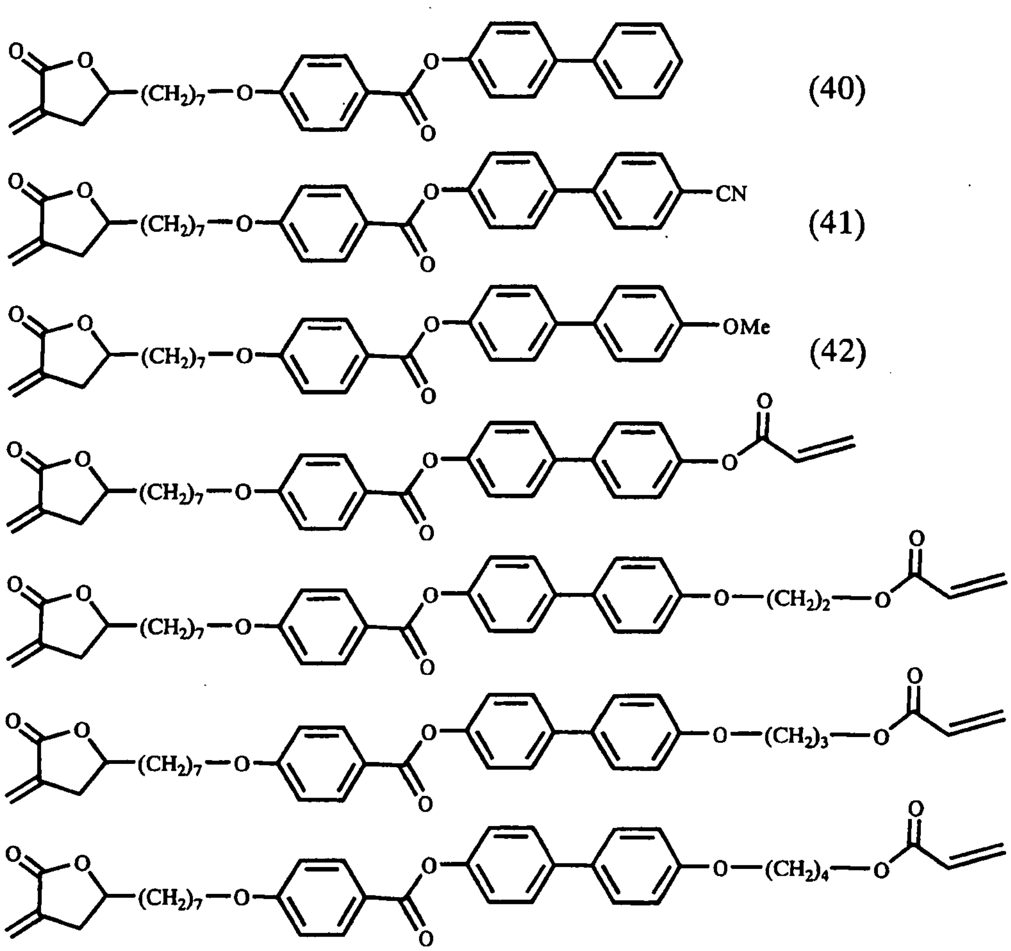

(40)

(41)

(42)

(43)

(44)

(45)

(46)

[Chemical Formula 13]

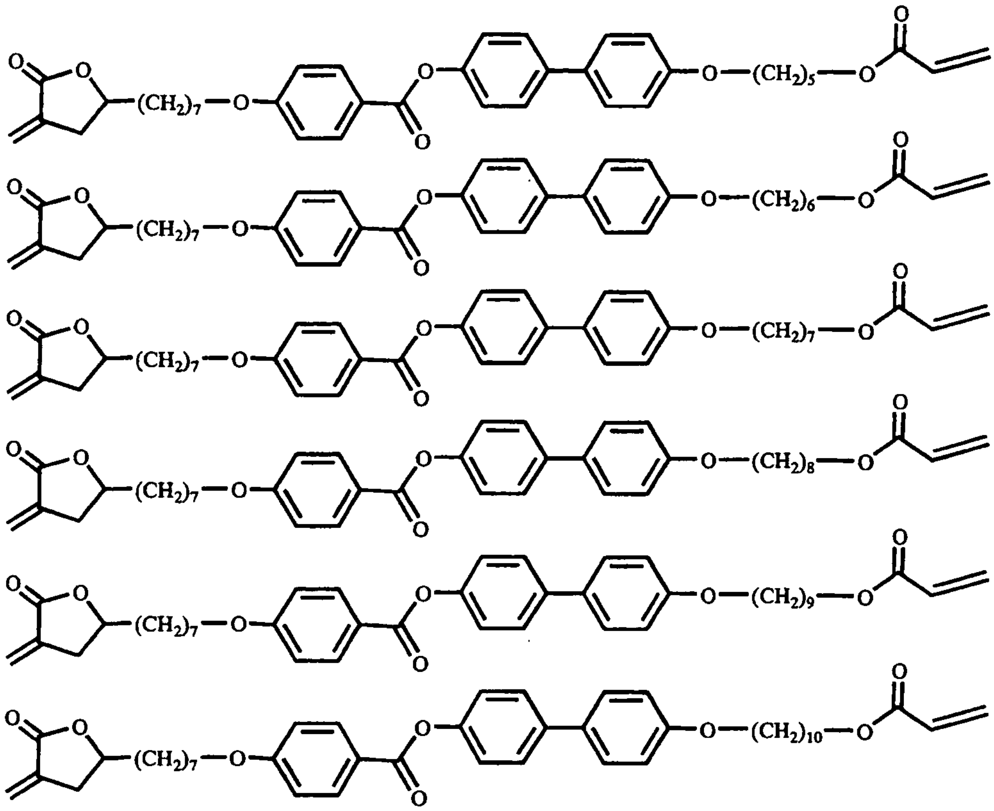

(47)

(48)

(49)

(50)

(51)

(52)

[Chemical Formula 14]

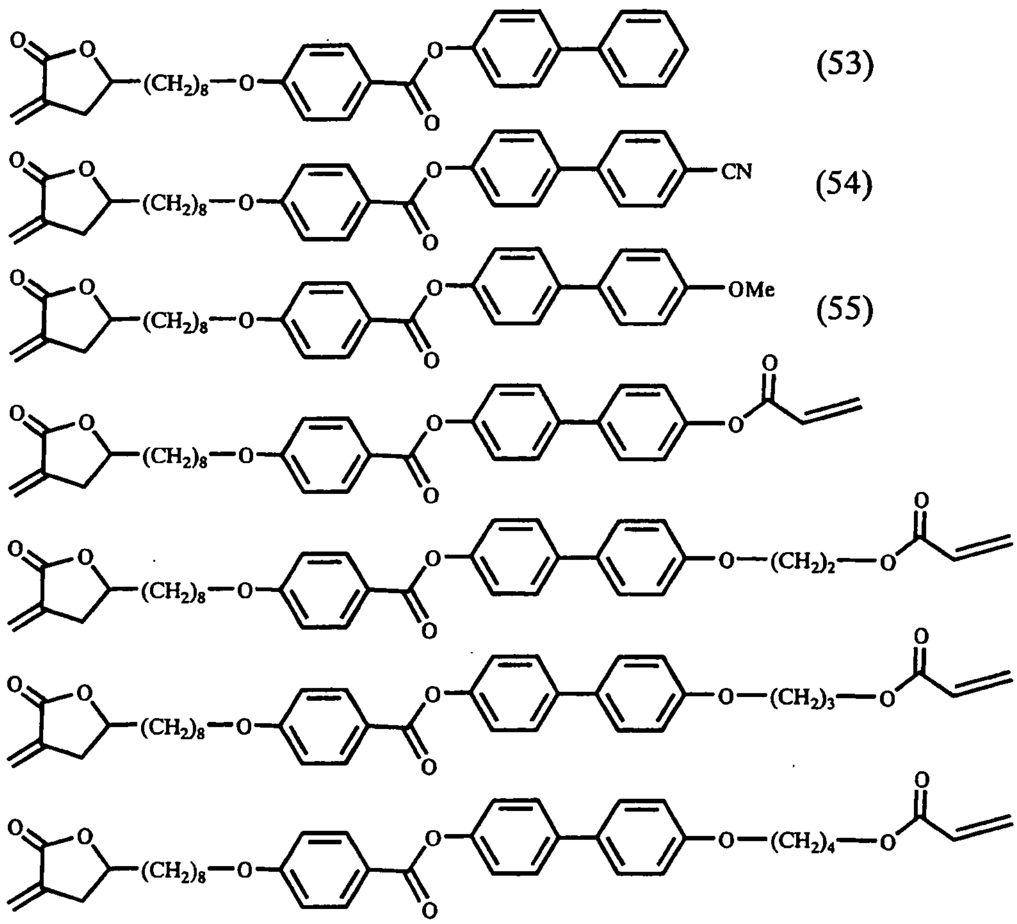

(53)

(54)

(55)

(56)

(57)

(58)

(59)

[Chemical Formula 15]

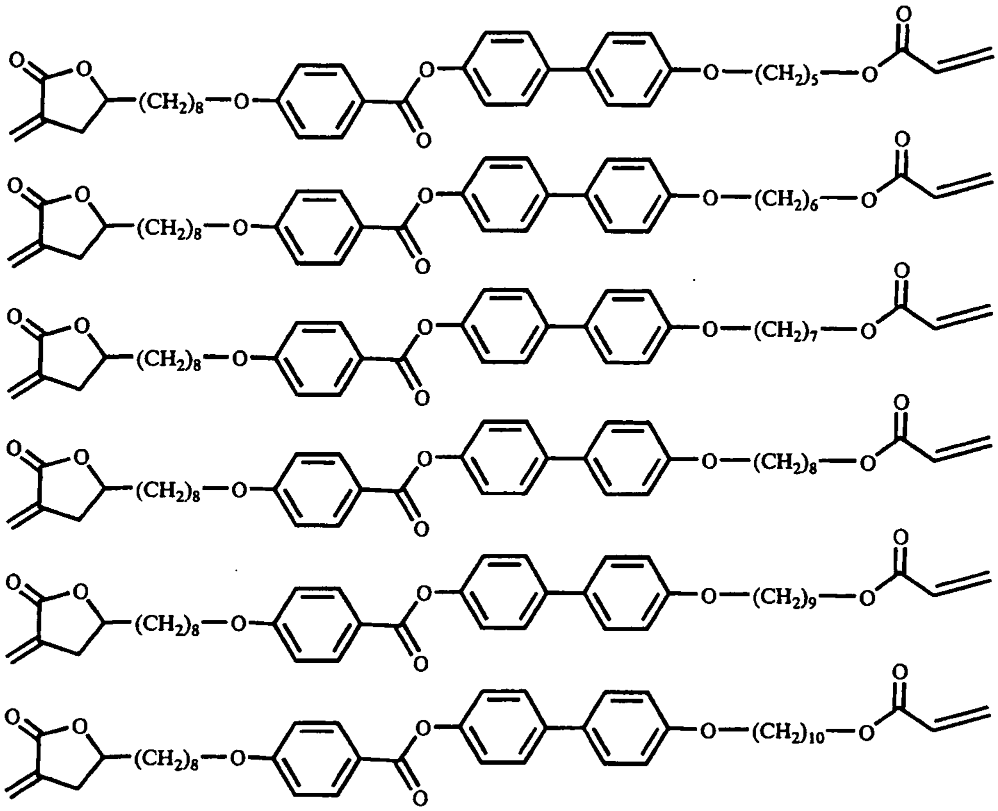

(60)

(61)

(62)

(63)

(64)

(65)

[Chemical Formula 16]

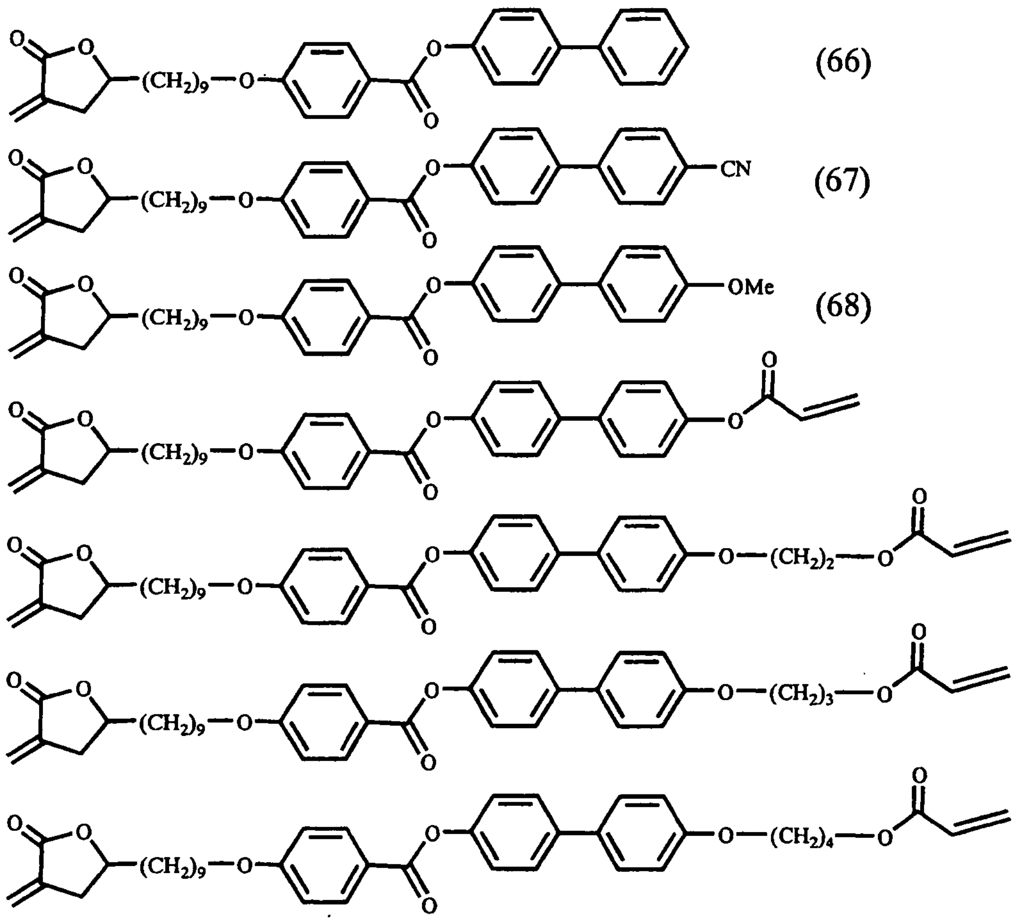

(66)

(67)

(68)

(69)

(70)

(71)

(72)

[Chemical Formula 17]

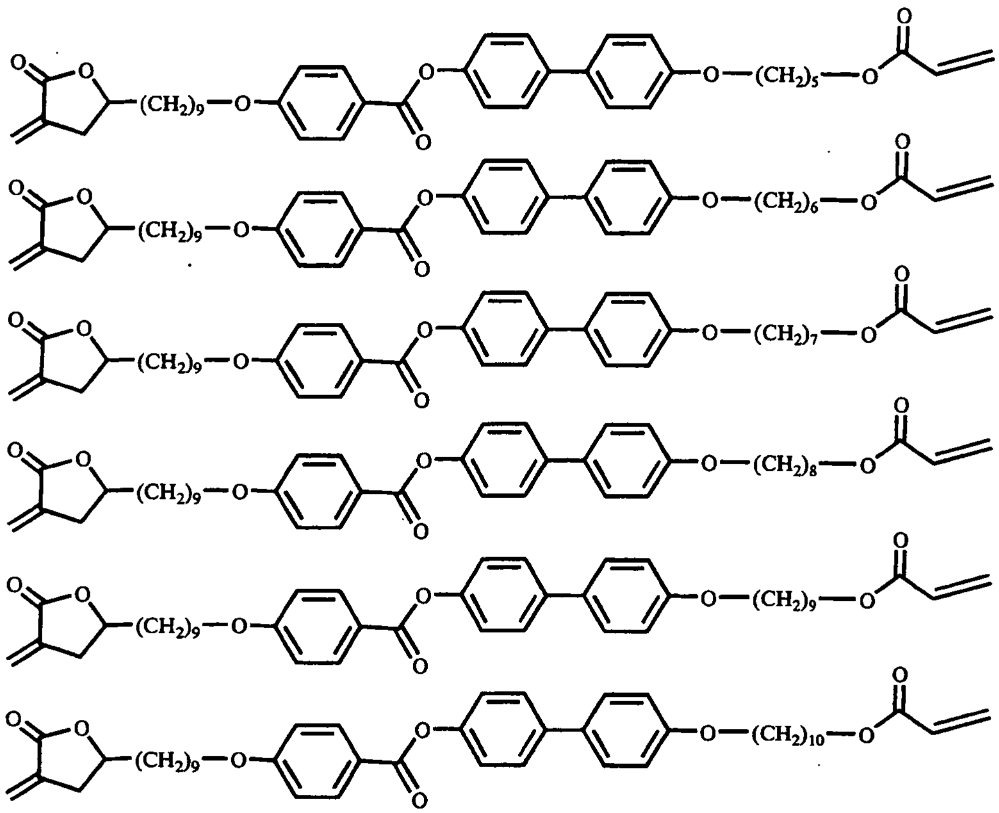

(73)

(74)

(75)

(76)

(77)

(78)

[Synthesis of polymerizable liquid crystal compounds]

The polymerizable liquid crystal compounds of the present invention can be synthesized through a combination of techniques in organic synthetic chemistry and no limitation is placed on the synthetic process.
The compound having an $\alpha$-methylene-$\gamma$-butyrolactone structure can be prepared by the technique expressed by the following synthetic scheme (A) and proposed by Talaga et al (P.Talaga, M.Schaeffer, C.Benezra and J.L.Stampf, Synthesis, 530(1990)). This technique is a process wherein 2-(bromomethyl)propenoic acid and an aldehyde or ketone are reacted by use of $SnCl_2$.
It will be noted that 2-(bromomethyl)propenoic acid can be obtained according to a process proposed by K.Ramarajan et al (K.Ramarajan, K.Kamalingam, D.J.O'Donnell and K.D.Berlin, Organic Synthesis, vol.61, 56-59 (1983)).
[Chemical Formula 18]

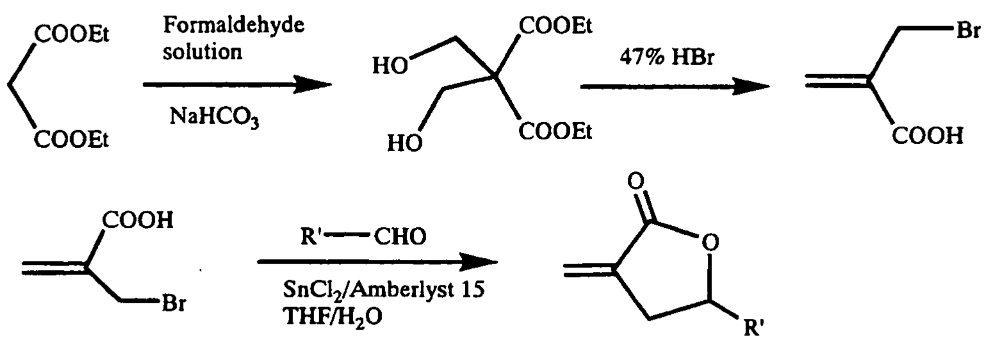


(A)

(In the formula, R' represents a monovalent organic group, and Amberlyst is a registered trade name of Rohm and Haas Company.)

With the reaction of 2-(bromomethyl)propenoic acid using $SnCl_2$, the α-methylene-γ-butyrolactone structure can be obtained by reaction with an acetal or ketal.

As an acetal or ketal, mention can be made of a dimethylacetal group, a diethylacetal group, a 1,3-dioxane group, a 1,3-dioxolane group or the like. The synthetic process and protective group (PG) are indicated in the following scheme (AA).

[Chemical Formula 19]

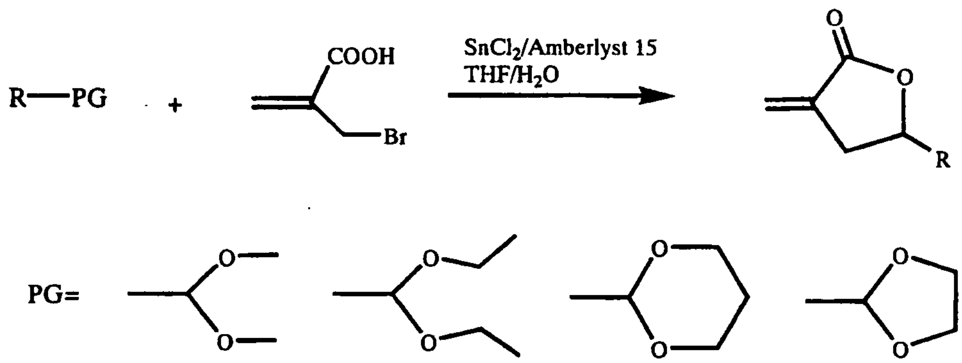


(AA)

The compound (intermediate) represented by the formula [2] can be prepared according to a technique of the following synthetic scheme (B) applied with the technique of the above synthetic scheme (A).

[Chemical Formula 20]

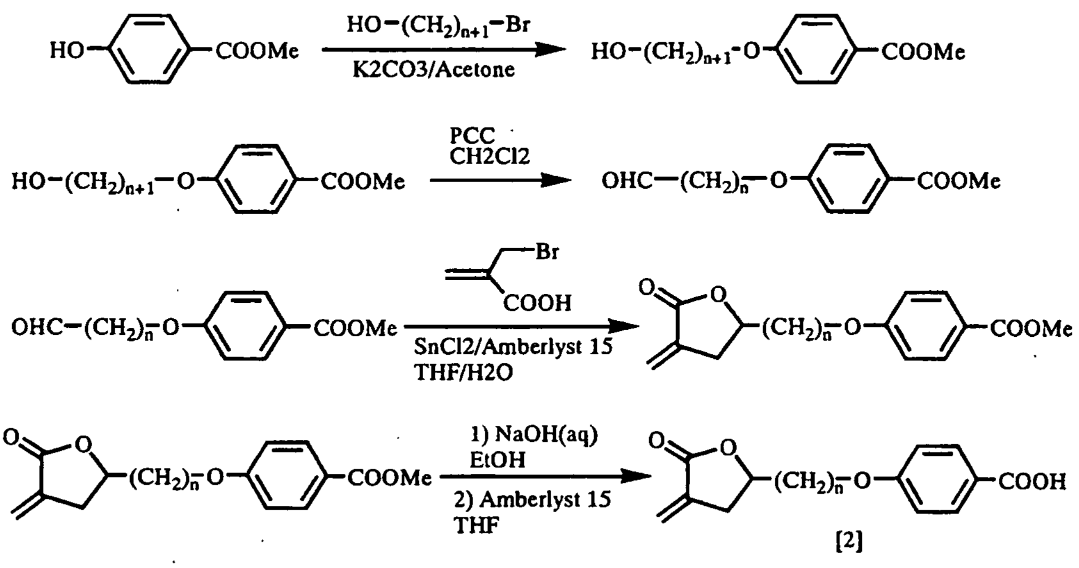


(B)

(In the formula, n has the same meaning as defined above.)

Next, as shown in synthetic scheme (C), the compound represented by the formula [2] and a phenolic compound are reacted for esterification, thereby obtaining the polymerizable liquid crystal compound represented by the formula [1].

[Chemical Formula 21]

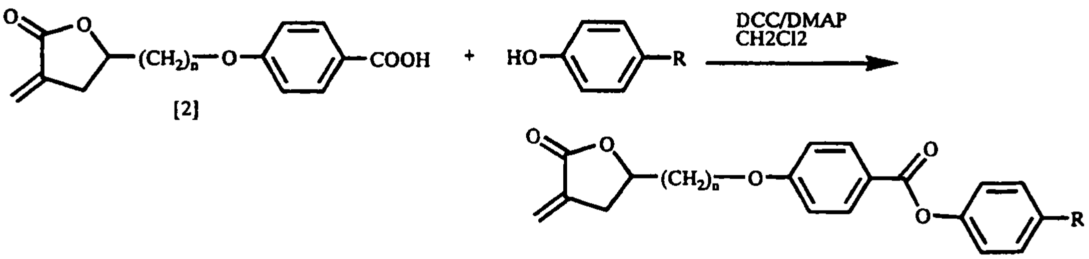


(C)

(In the formula, n and R, respectively, have the same meanings as defined before.)

The phenolic compounds of the synthetic scheme (C) wherein R is, respectively, [A-1] and [C-1] are commercially sold ones and are thus readily available.

The synthetic processes of compounds wherein R is, respectively, [B-1] and [B-2] are shown in the following synthetic schemes (D) and (E), respectively.

[Chemical Formula 22]

[B-1]

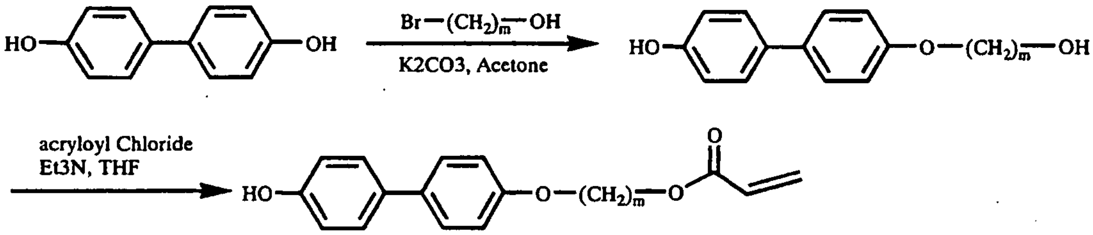


(D)

(In the formula, m has the same meaning as defined before.)

[Chemical Formula 23]

[B-2]

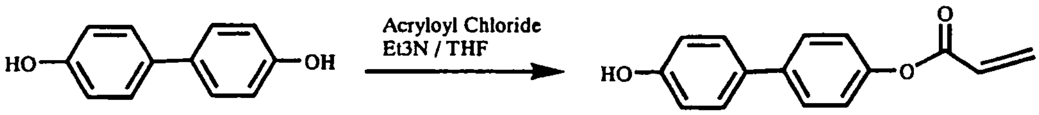


(E)

[Polymerizable liquid crystal composition]

The polymerizable liquid crystal composition of the present invention is one which includes at least one of the polymerizable liquid crystal compounds represented by the afore-indicated formula [1]. Where two or more of the polymerizable liquid crystal compounds represented by the formula [1] are used in the polymerizable liquid crystal composition, polymerizable liquid crystal compounds may be appropriately selected and mixed in arbitrary combinations.

With the polymerizable liquid crystal composition of the present invention, a compound having a liquid crystal structural moiety (hereinafter referred to as specified compound) may be admixed with the polymerizable liquid crystal compound of the formula [1]. The specified compounds to be mixed may be used in combination of a plurality thereof. In this connection, the specified compound may be either a compound showing liquid crystallinity or a compound showing no liquid crystallinity, and may have or may not have a polymerizable group such as an acryl group, a lactone ring or the like. The specified compound having a polymerizable group may be either monofunctional or polyfunctional.
Such a specific compound includes a compound that has no polymerizable group but shows liquid crystallinity, a compound that has no polymerizable group and does not show liquid crystallinity, a compound having a polymerizable group and showing liquid crystallinity, but other than the polymerizable liquid crystal compounds of the present invention, or a compound having a polymerizable group and showing no crystallinity.

The formulating ratio of the specified compound is not limited. However, if a specified compound to be mixed shows liquid crystallinity, the ratio is preferably at not larger than 1900 parts by weight, more preferably at not larger than 500 parts by weight, per 100 parts by weight of the polymerizable liquid crystal compound of the formula [1]. It will be noted that when it is taken into account that an optical film obtained from the polymerizable liquid crystal composition of the present invention is permitted to show a good chemical resistance, the amount of the specified compound showing liquid crystallinity is preferably at not larger than 900 parts by weight, more preferably not larger than 150 parts by weight, per 100 parts by weight of the polymerizable liquid crystal compound of the formula [1].
On the other hand, where a specified compound to be mixed shows no liquid crystallinity, the amount is preferably at not larger than 20 parts by weight, more preferably not larger than 5 parts by weight, per 100 parts by weight of the polymerizable liquid crystal compound of the formula [1].

Specific examples of the specified compound include those compounds represented by the following formulas (79) to (104), which are described in PCT Patent Publication No. WO06/115033 Pamphlet and PCT Patent Publication No. WO06/115112 Pamphlet, and nematic liquid crystals, ferroelectric liquid crystals, commercially available liquid crystal compositions and the like although not limited thereto.

[Chemical Formula 24]

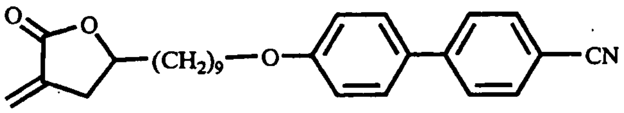
(79)

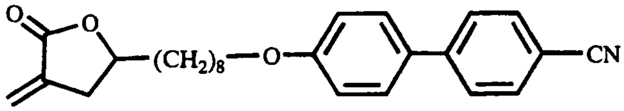
(80)

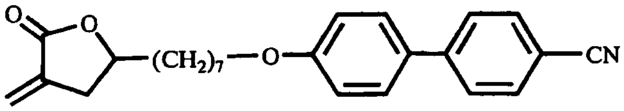
(81)

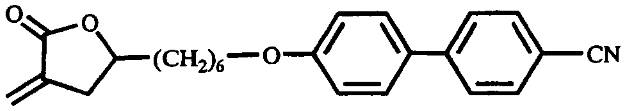
(82)

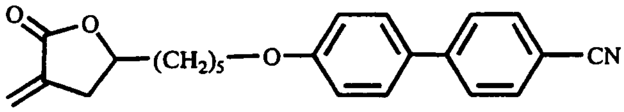
(83)

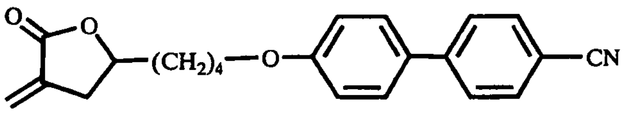
(84)

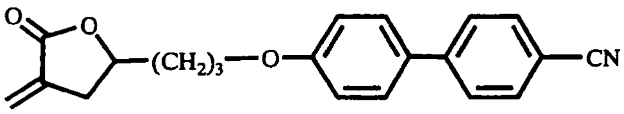
(85)

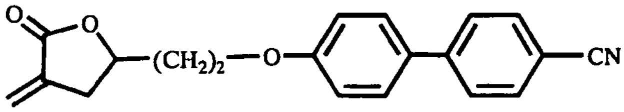
(86)

[Chemical Formula 25]

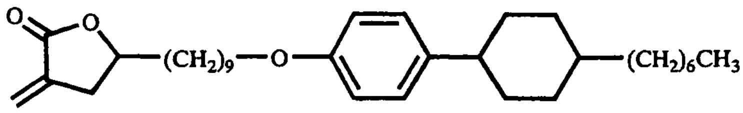

(87)

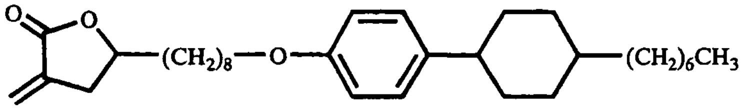

(88)

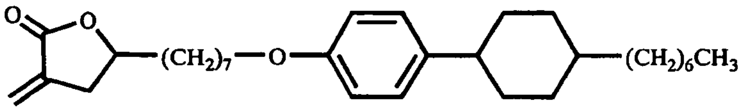

(89)

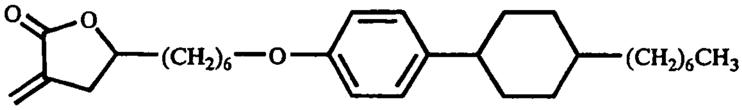

(90)

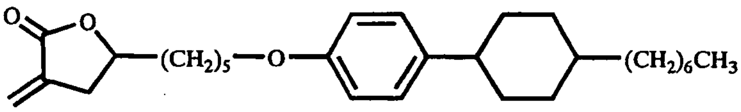

(91)

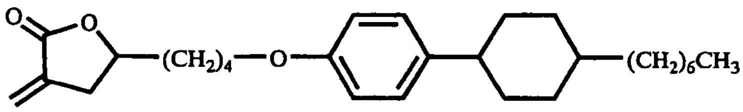

(92)

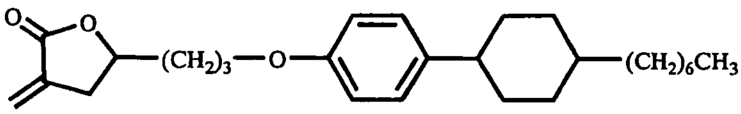

(93)

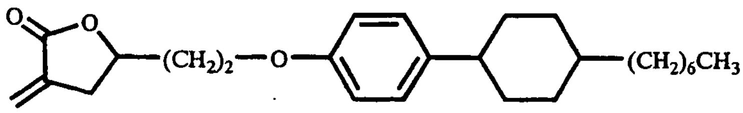

(94)

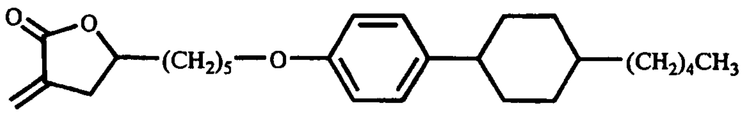

(95)

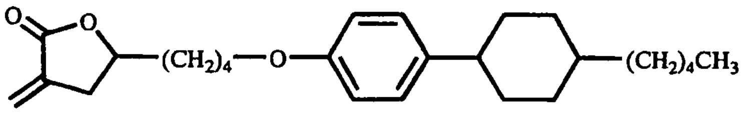

(96)

[Chemical Formula 26]

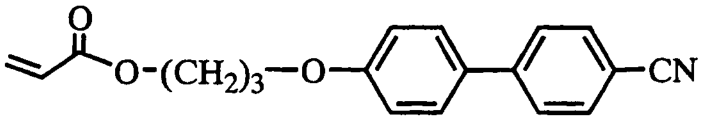
(97)

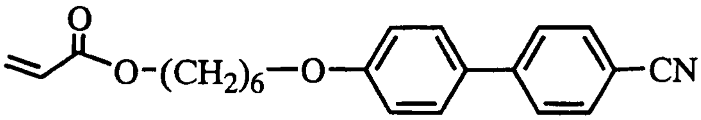
(98)

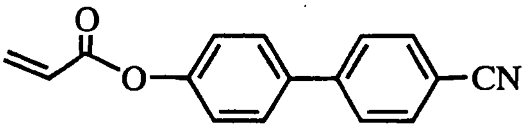
(99)

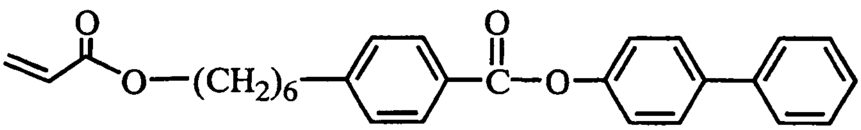
(100)

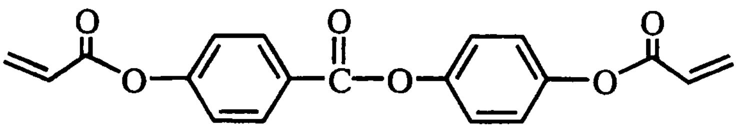
(101)

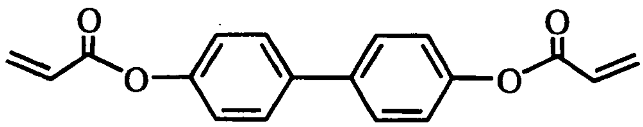
(102)

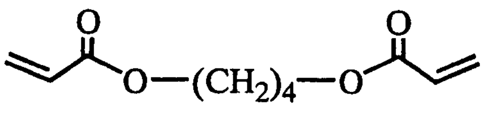
(103)

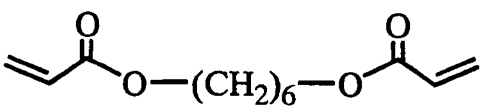
(104)

For the purpose of improving polymerization reactivity, photopolymerization initiators, thermal polymerization initiators or photosensitizers may be added to the polymerizable liquid crystal composition of the present invention. The photopolymerization initiators include, for example, benzoin ethers such as benzoin methyl ether, benzophenones such as benzophenone, acetophenones such as diethoxyacetophenone, benzyl ketals such as benzyl dimethyl ketal, and the like. These photopolymerization initiators may be used in combination of a plurality thereof.
The adding amount of the photopolymerization initiator is preferably at not larger than 5 parts by weight, more preferably from 0.5 to 2 parts by weight, per 100 parts by weight of the total amount of the polymerizable liquid crystal compounds represented by the formula [1] or the total amount of the polymerizable liquid crystal compound represented by the formula [1] and a specified compound having a polymerizable group and showing liquid crystallinity (both in combination are hereinafter referred to as total polymerizable liquid crystal compounds).

The thermal polymerization initiator includes, for example, 2,2'-azobisisobutyronitrile or the like. The thermal polymerization initiators may be used in combination of a plurality thereof, and the adding amount is preferably at not larger than 5 parts by weight, more preferably from 0.5 to 2 parts by weight, per 100 parts by weight of the total polymerizable liquid crystal compounds represented by the formula [1].
The photosensitizer includes, for example, an anthracene photosensitizer such as anthracene or the like. The photosensitizers may be used in combination of a plurality thereof. The adding amount is preferably not larger than 5 parts by weight per 100 parts by weight of the total polymerizable liquid crystal compounds.
It will be noted that the above photopolymerization initiator may be used in combination with at least one of the thermal

polymerization initiator and the photosensitizer.

For the purpose of improving storage stability, stabilizers may be added to the polymerizable liquid crystal composition of the present invention.

The stabilizer includes, for example, hydroquinone, hydroquinone monoalkyl ethers such as hydroquinone monomethyl ether, 4-t-butyl catechol, or the like. The stabilizers may be used in combination of a plurality thereof and the adding amount is preferably not larger than 0.1 parts by weight per 100 parts by weight of the total polymerizable liquid crystal compounds.

In order to improve adhesion to a substrate, adhesion promoting agents may be added to the polymerizable liquid crystal composition of the present invention.

The adhesion promoting agents include: chlorosilanes such as trimethylchlorosilane, dimethylvinylchlorosilane, methyldiphenylchlorosilane, chloromethyldimethylchlorosilane and the like; alkoxysilanes such as trimethylmethoxysilane, dimethyldiethoxysilane, methyldimethoxysilane, dimethylvinylethoxysilane, diphenyldimethoxysilane, phenyltriethoxysilane and the like; silazanes such as hexamethyldisilazane, N,N'-bis(trimethylsilyl)urea, dimethyltrimethylsilylamine, trimethylsilylimidazole and the like; silanes such as vinyltrichlorosilane, γ-chloropropyltrimethoxysilane, γ-aminopropyltriethoxysilane, γ-methacryloxypropyltrimethoxysilane, γ-glycidoxypropyltrimethoxysilane, γ-(N-piperidinyl)propyltrimethoxysilane and the like; heterocyclic compounds such as benzotriazole, benzimidazole, indazole, imidazole, 2-mercaptobenzimidazole, 2-mercaptobenzothiazole, 2-mercaptobenzoxazole, urazole, thiouracil, mercaptoimidazole, mercaptopyrimidine and the like; and urea compounds such as 1,1-dimethylurea, 1,3-dimethylurea and the like, and thiourea compounds.

The adhesion promoting agents may be used in combination of a plurality thereof and the adding amount is preferably not larger than 1 part by weight per 100 parts by weight of the total polymerizable liquid crystal compounds.

Further, organic solvents may be added to the polymerizable liquid crystal composition of the present invention in order to control the viscosity thereof. In this case, the composition may not show liquid crystallinity in a condition where organic solvents are contained.

Examples of the organic solvent include ethers such as tetrahydrofuran, dioxane and the like, aromatic hydrocarbons such as benzene, toluene, xylene and the like, polar solvents such as N,N-dimethylformamide, N-methyl-2-pyrrolidone and the like, esters such as ethyl acetate, butyl acetate, ethyl lactate and the like, alkoxyesters such as methyl 3-methoxypropionate, methyl 2-methoxypropionate, ethyl 3-methoxypropionate, ethyl 2-methoxypropionate, ethyl 3-ethoxypropionate, ethyl 2-ethoxypropionate and the like, glycol dialkyl ethers such as ethylene glycol dimethyl ether, propylene glycol dimethyl ether and the like, diglycol dialkyl ethers such as diethylene glycol dimethyl ether, diethylene glycol diethyl ether, diethylene glycol methyl ethyl ether, dipropylene glycol dimethyl ether and the like, glycol monoalkyl ethers such as ethylene glycol monomethyl ether, ethylene glycol monoethyl ether, propylene glycol monomethyl ether, propylene glycol monoethyl ether and the like, diglycol monoalkyl ethers such as diethylene glycol monomethyl ether, diethylene glycol monoethyl ether, dipropylene glycol monomethyl ether, dipropylene glycol monoethyl ether and the like, glycol monoalkyl ether esters such as propylene glycol monomethyl ether acetate, carbitol acetate, ethyl cellosolve acetate and the like, and ketones such as cyclohexanone, methyl ethyl ketone, methyl isobutyl ketone, 2-heptanone and the like. These organic solvents may be used singly or in combination of two or more.

Of these, it is preferred from the standpoint of safety to global and working environments to use propylene glycol monomethyl ether, propylene glycol monomethyl ether acetate and ethyl lactate.

It will be noted that the amount of the solvent is preferably about 60 to 95 wt% of the polymerizable liquid crystal composition.

For the purpose of improving affinity for a substrate, surface active agents may be added to the polymerizable liquid crystal composition of the present invention. As a surface active agent, mention is made of fluorine-based surface active agents, silicone surface active agents, nonionic surface active agents and the like although not limited thereto.

The fluorine-based surface active agent is preferred because of the high affinity-improving effect on substrates.

Specific examples of the fluorine-based surface active agent include (as a commercial name indicated hereinbelow) F Top EF301, EF303 and EF 352 (made by To-chem Products Corporation), Megafac F171, F173 and R-30 (made by DIC corporation), Fluorad FC430, FC431 (made by Sumitomo 3M Limited), Asahi Guard AG710, Surflon S-382, SC101, SC102, SC103, SC104, SC105, SC106 (Asahi Glass Co., Ltd.) and the like although not limited thereto. It will be noted that the surface active agents may be used in combination of a plurality thereof.

Preferred examples of the polymerizable liquid crystal composition of the present invention include a liquid crystal composition which includes 100 parts by weight of a polymerizable liquid crystal compound represented by the formula [1] and not larger than 5 parts by weight of a photopolymerization initiator, a liquid crystal composition which includes 100 parts by weight of a polymerizable liquid crystal compound represented by the formula [1], not larger than 20 parts by weight of a specified compound showing no liquid crystallinity, and not larger than 5 parts by weight of a photopolymerization initiator, and a liquid crystal composition which includes 100 parts by weight of a polymerizable liquid crystal compound represented by the formula [1], not larger than 400 parts by weight of a specified compound showing liquid crystallinity, and not larger than 5 parts by weight of a photopolymerization initiator.

The polymerizable liquid crystal composition set forth above can be conveniently used as a composition for forming an alignment film or a coating solution.

The manner of preparing the polymerizable liquid crystal composition of the present invention is not limited. The ingredients making up of the polymerizable liquid crystal composition may be mixed at once or may be mixed successively. For the successive mixing, the respective ingredients may be added in an arbitrary order.

It will be noted that if a plurality of compounds are used for a single ingredient, a mixture of the plurality of compounds obtained by preliminary mixing and other ingredients may be mixed together, or individual compounds may be, respectively, mixed with other ingredients.

The polymerizable liquid crystal composition of the present invention preferably shows an enantiotropic liquid crystal phase at room temperature (20 to 40°C herein and whenever it appears hereinafter) for the reason that when an optical anisotropic material is prepared, unintended thermal polymerization is avoided from occurring in the course of photopolymerization in a liquid crystal condition and the molecules are readily allowed to be fixed in a uniform alignment state. Where an organic solvent is contained in the polymerizable liquid crystal composition, it is preferred that an enantiotropic liquid crystal phase is shown at room temperature upon removal of the solvent.

[Polymers and Films]

Photoirradiation or thermal treatment applied on the thus illustrated polymerizable liquid crystal composition of the present invention results in the formation of a polymer.

Under conditions where the polymerizable liquid crystal composition is sandwiched between two substrates or where the polymerizable liquid crystal composition is coated on a substrate by spin coating or casting, photoirradiation treatment results in the formation of a film.

The substrate used may be glass, quartz, plastic sheets, color filters, or plastic films such as triacetyl cellulose (TAC) or the like. It will be noted that as one of the two substrates, there may be used glass, plastic sheets, plastic films and stainless steel on which a functional thin film such as ITO or the like has been formed, or belts or drums on which a metal such as chromium, aluminium or the like has been plated or vacuum deposited.

An employed substrate is preferably subjected to alignment treatment for the purpose of improving the alignment of the resulting film. For the alignment treatment, there may be used those methods appropriately selected from known methods, including a method wherein an alignment material including a polyimide precursor, a polyimide, polyvinyl cinnamate or the like is subjected to alignment treatment by coating and then rubbing or irradiation with polarized UV light, a method of forming an oblique vacuum deposition film of silicon dioxide, a method for forming a Langmuir film and the like.

In the method of sandwiching the polymerizable liquid crystal composition between two substrates, there is formed a cell wherein a space is established between two substrates such as spacers, after which a polymerizable liquid crystal composition is charged into the cell by a method of utilizing a capillary action or a method of depressurizing the space in the cell, followed by irradiation of light for polymerization.

A simpler method is one wherein a polymerizable liquid crystal composition is placed on a substrate provided with a spacer or the like, putting another substrate thereon to form a cell, and irradiating light thereon for polymerization.

Although the polymerizable liquid crystal composition used may be fluid or may be fluidized by heating after placing on the substrate, it is necessary that the polymerizable liquid crystal composition be fluidized prior to putting another substrate on top thereof.

In the method of coating a polymerizable liquid crystal composition, a heating step using a hot plate or the like may be added, if necessary, in the middle between the coating step of the polymerizable liquid crystal composition and the step of polymerization with light or heat. Especially, when the polymerizable liquid crystal composition (coating solution) contains an organic solvent, this step is effective for removing the organic solvent from the composition.

In any of the above-mentioned methods, there can be obtained an aligned film having optical anisotropy by polymerizing the polymerizable liquid crystal composition that is in a condition of showing a liquid crystal phase.

In order to obtain a polymer in a multi-domain state having a different alignment in every adjacent domain, there are used a method of multi-domain conversion in the course of polymerization, and a multi-domain conversion method for the alignment treatment of a substrate.

The multi-domain conversion method using a polymerization technique is one wherein UV light is exposed through a mask to a polymerizable liquid crystal composition in a liquid crystal state to form polymerized domains and remaining domains are polymerized in an isotropic liquid state.

The multi-domain conversion method for the alignment treatment of a substrate is one including a method wherein an alignment material formed on a substrate is rubbed through a mask or a method wherein UV light is irradiated through a mask.

According to these methods, there is obtained a multi-domain substrate wherein the rubbed domains and the domains irradiated with UV light correspond to an aligned portion and the other is an untreated portion. The polymerizable liquid

crystal composition formed on the multi-domain substrate undergoes multi-domain conversion by the influence of an alignment material layer.
It will be noted that aside from the above alignment treatment methods, there may also be used methods using an electric field and a magnetic field.

Using the polymerizable liquid crystal composition of the present invention, there can be obtained a film having optical anisotropy, and this film can be conveniently used as a polarizer plate, a phase difference plate and the like. Additionally, the film has good transparency at high temperatures and can be suitably utilized as electronic devices, such as on-vehicle display devices and the like, used in high-temperature environment.

## EXAMPLES

The present invention is more particularly described by way of synthetic examples, examples and comparative examples. The present invention should not be construed as limited to the following examples. It will be noted that the measuring methods and measuring conditions of physical properties in the examples are as illustrated below.

[1] NMR

A compound was dissolved in deuterated chloroform ($CDCl_3$) or deuterated dimethylsulfoxide (DMSO-d6) and $^1$H-NMR at 300 MHz was measured by use of a nuclear magnetic resonance apparatus (made by Diol Company).

[2] Observation of liquid phase

Identification of a liquid phase was performed by heating a sample on a hot stage (MATS-2002S, made by Tokaihit Company) and observing the phase by use of a polarizing microscope (made by Nikon Corporation). The phase transition temperature was measured by using a differential scanning thermal analyzer (DSC3100SR) (hereinafter referred to as DSC) made by MAC Science Co., Ltd., under conditions of a scanning rate of 10°C/minute.

[2] Haze value

The haze value of a film was measured by use of Spectral Haze Mater (TC-1800H), made by Tokyo Denshoku Co., Ltd.

[3] Retardation value of film

The retardation value was measured at a wavelength of 590 nm by use of a retardation measuring apparatus (RETS-100, made by Otsuka Electronics Co., Ltd.)

Synthetic Example 1

Synthesis of polymerizable liquid crystal compound (E3)

5.0 g (25.6 mmol) of 4-cyano-4'-hydroxybiphenol, 4.6 g (25.6 mmol) of 6-bromo-1-hexanol, 7.0 g (50 mmol) of potassium carbonate and 50 ml of acetone were added to a 100 ml round bottom flask equipped with a condenser to provide a mixture, followed by reaction at 64°C for 24 hours under stirring. After completion of the reaction, the solvent was distilled off under reduced pressure to obtain a yellow wet solid. Thereafter, this solid was mixed with 70 ml of water, to which 50 ml of diethyl ether was added for extraction. The extraction was repeated three times.
Anhydrous magnesium sulfate was added to a separated organic phase for drying and after filtration, the solvent was distilled off under reduced pressure to obtain a yellow solid. This solid was dissolved in 3 ml of ethyl acetate and purified by silica gel column chromatography (column: silica gel 60, 0.063-0.200 mm, made by Merck & Co., eluate: hexane/ethyl acetate = 1/1). The solvent was distilled off from the resulting solution to obtain 6.9 g of a white solid. The solid was subjected to measurement of NMR with the results shown below. From the results, it was confirmed that the white solid was made of an intermediate compound (A1) shown in the following synthetic scheme (yield: 91%).

$^1$H-NMR(DMSO-d6) δ: 1.26(m, 6H), 1.69(m, 2H), 3.37(t, 2H), 4.03(t, 2H), 7.06(d, 2H), 7.69(d, 2H), 7.85(m, 4H).

[Chemical Formula 27]

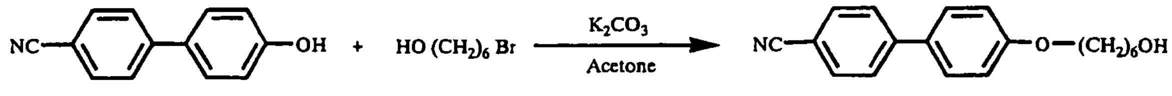


(A1)

Next, 2.2 g (10.0 mmol) of pyridinium chlorochromate (hereinafter abbreviated as PCC) and 30.0 ml of $CH_2Cl_2$ were added to a 200 ml three-necked flask equipped with a condenser and mixed under stirring, in which a solution of 2.95 g (10.0 mmol) of the intermediate compound (A1) obtained above dissolved in $CH_2Cl_2$ (50.0 ml) was dropped, followed by further stirring at 40°C for 0.5 hours. Thereafter, 90 ml of diethyl ether was added to the solution, from which an oily matter attached to the wall of the flask was removed, and subjected to filtration under reduced pressure, followed by distilling off the solvent under reduced pressure to obtain a dark green wet solid.
This solid was dissolved in 3 ml of ethyl acetate and purified by silica gel chromatography (column: silica gel 60, 0.063-0.200 mm, made by Merck & Co., eluate: hexane/ethyl acetate = 1/1). The solvent was distilled off from the resulting solution to obtain 2.8 g of a colorless solid. This solid was subjected to measurement of NMR with the results shown below. From the results, it was confirmed that this colorless solid was made of an intermediate compound (B1) (yield: 93%) shown in the following synthetic scheme.

$^1$H-NMR($CDCl_3$) δ: 1.84(m, 6H), 2.50(m, 2H), 4.02(m, 2H), 6.99(d, 2H), 7.53(d, 2H), 7.91(m, 4H), 9.80(s, 1H).

[Chemical Formula 28]

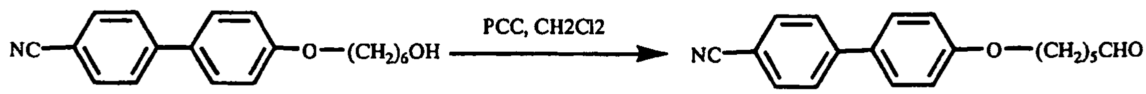


(B1)

Finally, 3.0 g (10.0 mmol) of the intermediate compound (B1) obtained above, 1.65 g (10.0 mmol) of 2-(bromomethyl)acrylic acid, 1.6 g of Amberlyst (registered trade name) 15 (commercial name of Rohm & Haas Company), 16.0 ml of tetrahydrofuran (THF), 1.9 g (10.0 mmol) of tin (II) chloride and 4.0 ml of pure water were added to a 50 ml round bottom flask equipped with a condenser to provide a mixture, followed by stirring for reaction at 70°C for 7 hours. After completion of the reaction, the reaction solution was filtered under reduced pressure and mixed with 30 ml of pure water, to which 50 ml of diethyl ether was added for extraction. The extraction was performed three times.
Anhydrous magnesium sulfate was added for drying to the organic phase obtained after the extraction and filtered under reduced pressure, and the solvent was distilled off from the resulting solution to obtain a yellow solid. This solid was dissolved in 2 ml of ethyl acetate and purified by silica gel chromatography (column: silica gel 60, 0.063-0.200 mm, made by Merck & Co., eluate: hexane/ethyl acetate = 2/1). The solvent was distilled off from the resulting solution to obtain 1.5 g of a white solid. This solid was subjected to measurement of NMR, with the result that it was confirmed that this white solid was made of the intended polymerizable liquid crystal compound (E3) (yield: 41%).

$^1$H-NMR($CDCl_3$) δ: 1.57(m, 6H), 1.85(m, 2H), 2.60(m, 1H), 3.05(m, 1H), 4.01(t, 2H), 4.54(m, 1H), 5.63(m, 1H), 6.23(m, 1H), 7.00(d, 2H), 7.52(d, 2H), 7.68(m, 4H).

The results of observation of the crystallinity of this polymerizable liquid crystal compound revealed that it converted to an isotropic liquid at 84°C, and underwent phase transition into a liquid crystal phase (nematic phase) at 61°C upon temperature drop.

[Chemical Formula 29]

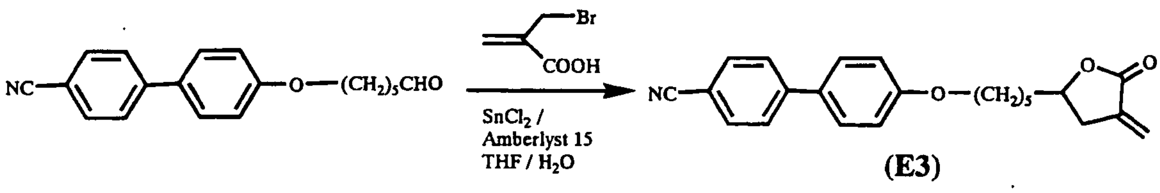


Synthetic Example 2

Synthesis of polymerizable liquid crystal compound (E2)

[Chemical Formula 30]

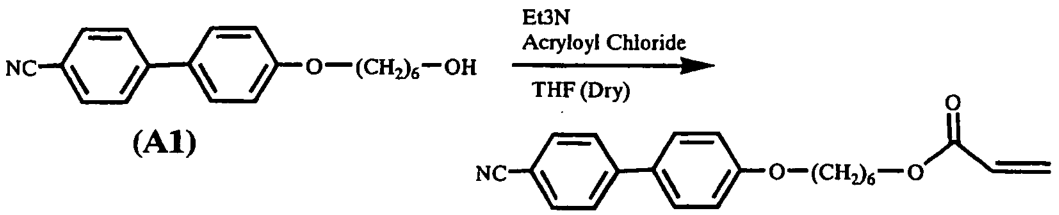

(E2)

3.0 g of the intermediate compound (A1) obtained in the same manner as in Synthetic Example 1 was dissolved in 10 ml of THF along with 1.5 ml of triethylamine and a small amount of 3,5-di(tert-butyl)-4-hydroxytoluene (BHT) and stirred at room temperature, in which a solution of 0.9 ml of acryloyl chloride in 10 ml of THF was dropped over 15 minutes under cooling on a water bath. After the dropping, the reaction solution was stirred for 30 minutes, after which the water bath was removed and stirring was continued overnight while returning to room temperature, followed by filtration of a precipitated TEA hydrochloride salt. About 3/4 of the THF was distilled off from the resulting filtrate, to which 50 ml of methylene chloride was added. The resulting organic phase was washed successively with 50 ml of a saturated sodium hydrogen carbonate aqueous solution, 50 ml of 0.5N-HCl and 50 ml of a saturated saline solution, and dried over magnesium sulfate, followed by distilling off the solvent to obtain a product. After recrystallization with methanol, 1.7 g of compound (E2) was obtained.

$^1$H-NMR($CDCl_3$) δ: 1.50(m, 4H), 1.73(m, 2H), 1.85(m, 2H), 4.05(t, 2H), 4.20(t, 2H), 5.82(d, 1H), 6.15(m, 1H), 6.41 (d, 1H), 6.99(d, 2H), 7.55(d, 2H), 7.66(m, 4H)

Synthetic Example 3

Synthesis of polymerizable liquid crystal compound (E1)

[Chemical Formula 31]

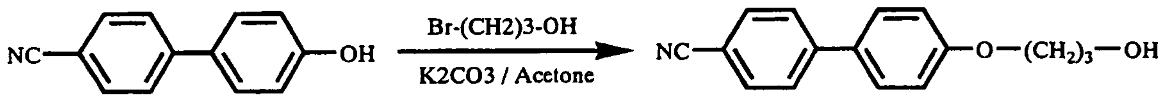


(A2)

9.8 g (50.0 mmol) of 4-cyano-4'-hydroxybiphenol, 7.0 g (50.0 mmol) of 3-bromo-1-propanol, 13.8 g (100 mmol) of potassium carbonate and 150 ml of acetone were added to a 500 ml round bottom flask equipped with a condenser to provide a mixture, followed by reaction at 64°C for 48 hours under stirring. After completion of the reaction, the solvent was distilled off under reduced pressure to obtain a yellow wet solid. Thereafter, this solid was mixed with 140 ml of water, to which 100 ml of diethyl ether was added for extraction. The extraction was repeated three times. Anhydrous magnesium sulfate was added to the separated organic phase for drying and the solvent was distilled off under reduced pressure after filtration to obtain a yellow solid. This solid was purified by recrystallization with a mixed solvent of hexane/ethyl acetate = 2/1 to obtain 8.7 g of a white solid. This solid was subjected to measurement of NMR with the results shown below. From the results, it was confirmed that the white solid was made of an intermediate compound (A2) (yield: 70%).

$^1$H-NMR($CDCl_3$) δ: 2.09(m, 2H), 3.90(t, 2H), 4.20(t, 2H), 6.99(d, 2H), 7.52(d, 2H), 7.66(m, 4H).

[Chemical Formula 32]

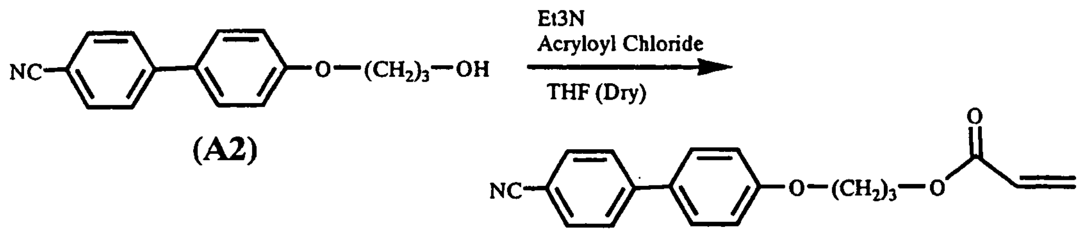


(A2)

(E1)

12.0 g of the intermediate compound (A2) obtained above was dissolved in 40 ml of THF along with 7.7 ml of triethylamine and a small amount of BHT and stirred at room temperature, in which a solution of 4.6 ml of acryloyl chloride in 40 ml of THF was dropped over 15 minutes under cooling on a water bath. After the dropping, the reaction solution was stirred for 30 minutes, after which the water bath was removed and stirring was continued overnight while returning to room temperature, followed by filtration of a precipitated TEA hydrochloride salt. About 3/4 of the THF was distilled off from the resulting filtrate, to which 50 ml of methylene chloride was added. The resulting organic phase was washed successively with 50 ml of a saturated sodium hydrogen carbonate aqueous solution, 50 ml of 0.5N-HCl and 50 ml of a saturated saline solution, and dried over magnesium sulfate, followed by distilling off the solvent to obtain a product. After recrystallization with ethanol, 6.0 g of compound (E1) was obtained.

$^1$H-NMR($CDCl_3$) δ: 2.20(m, 2H), 4.10(t, 2H), 4.40(t, 2H), 5.81(d, 1H), 6.15(m, 1H), 6.41(d, 1H), 6.99(d, 2H), 7.55 (d, 2H), 7.66(m, 4H).

Example 1

Synthesis of polymerizable liquid crystal composition (Z1)

(1) Synthesis of compound (P1)

[Chemical Formula 33]

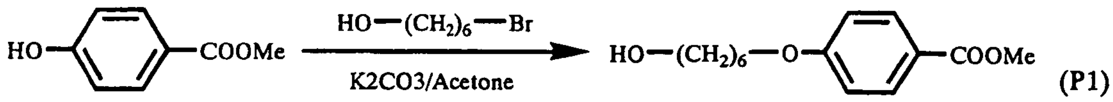


7.61 g (50.0 mmol) of methyl 4-hydroxybenzoate, 9.1 g (50.0 mmol) of 6-bromo-1-hexanol, 13.8 g (100 mmol) of potassium carbonate and 70 ml of acetone were added to a 200 ml round bottom flask equipped with a condenser to provide a mixture, followed by reaction under stirring at 64°C for 24 hours. After completion of the reaction, the reaction solution was filtered under reduced pressure and the solvent was distilled off under reduced pressure to obtain a yellow wet solid. This solid was purified by silica gel column chromatography (column: silica gel 60, 0.063-0.200 mm, made by Merck & Co., eluate: hexane/ethyl acetate = 1/1). The solvent was distilled off from the resulting solution to obtain 11.3 g of a white solid. This solid was subjected to measurement of NMR with the results shown below. From the results, it was confirmed that this white solid was made of the intermediate compound (P1) (yield: 90%).

$^1$H-NMR($CDCl_3$) δ: 1.3-1.7(m, 8H), 3.67(m, 2H), 3.88(s, 3H), 4.03(t, 2H), 6.91(d, 2H), 7.99(d, 2H).

(2) Synthesis of compound (Q1)

[Chemical Formula 34]

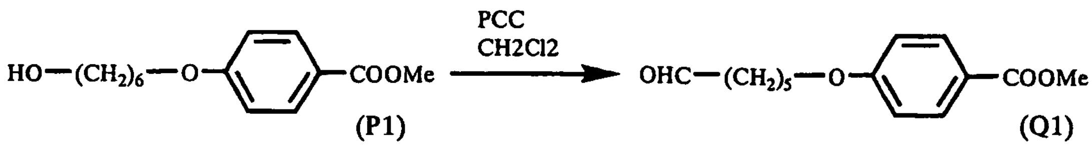


Next, 2.2 g (10.0 mmol) of PCC and 15.0 ml of $CH_2Cl_2$ were placed in a 100 ml three-necked flask equipped with a condenser and mixed under stirring, in which a solution, dissolved in $CH_2Cl_2$ (15.0 ml), of 2.5 g (10.0 mmol) of the intermediate compound (P1) obtained in the same manner as set out above was dropped, followed by further stirring at room temperature for 6 hours. Thereafter, 90 ml of diethyl ether was added to the solution, from which an oily matter attached to the flask wall was removed, and filtered under reduced pressure, after which the solvent was distilled off under reduced pressure to obtain a dark green wet solid.
This solid was purified by silica gel column chromatography (column: silica gel 60, 0.063-0.200 mm, made by Merck & Co., eluate: hexane/ethyl acetate = 2/1). The solvent was distilled off from the resulting solution to obtain 1.3 g of a colorless solid. This solid was subjected to measurement of NMR with the results shown below. From the results, it was confirmed that this colorless solid was made of the intermediate compound (Q1) (yield: 50%).

$^1$H-NMR($CDCl_3$) δ: 1.3-1.8(m, 8H), 2.49(t, 2H), 3.88(s, 3H), 3.99(t, 2H), 6.87(d, 2H), 7.99(d, 2H), 9.78(s, 1H).

(3) Synthesis of compound (R1)

[Chemical Formula 35]

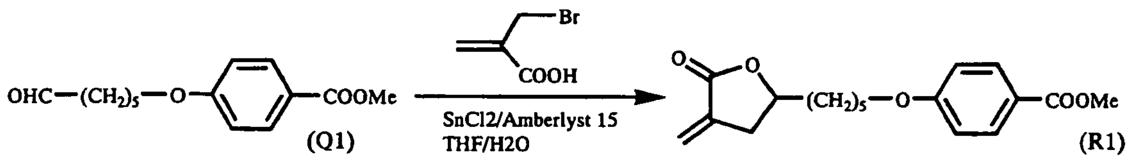


(Q1) (R1)

Next, 1.25 g (5.0 mmol) of the intermediate compound (Q1) obtained above, 0.83 g (5.0 mmol) of 2-(bromomethyl)acrylic acid, 0.8 g of Amberlyst (registered trade name) 15 (commercial name of Rohm & Haas Company), 8.0 ml of THF, 0.95 g (5.0 mmol) of tin (II) chloride and 2.0 ml of pure water were added to a 50 ml round bottom flask equipped with a condenser to provide a mixture, followed by stirring for reaction at 70°C for 5 hours. After completion of the reaction, the reaction solution was filtered under reduced pressure and mixed with 40 ml of pure water, to which 50 ml of diethyl ether was added for extraction. The extraction was performed three times.
Anhydrous magnesium sulfate was added for drying to the organic phase obtained after the extraction and filtered under reduced pressure, and the solvent was distilled off from the resulting solution to obtain 1.5 g of a colorless solid. This solid was subjected to measurement of NMR with the results shown below. From the results, it was confirmed that this colorless solid was made of the intermediate compound (R1) (yield: 94%).

$^1$H-NMR(DMSO-d6) δ: 1.3-1.8(m, 8H), 2.62(m, 1H), 3.04(s, 1H), 3.81(s, 3H), 4.05(t, 2H), 4.54(m, 1H), 5.70(s, 1H), 6.01(s, 1H), 7.03(d, 2H), 7.89(d, 2H).

(4) Synthesis of compound (Z1)

[Chemical Formula 36]

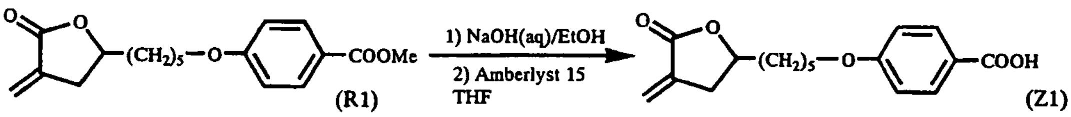


(R1) (Z1)

35 ml of ethanol, 1.5 g (4.7 mmol) of the compound (R1) obtained above and 5 ml of a 10% sodium hydroxide aqueous solution were added to a 100 ml round bottom flask equipped with a condenser to provide a mixture, followed by reaction under stirring at 85°C for 3 hours. After completion of the reaction, 300 ml of water and the reaction solution were added to a 500 ml beaker and stirred at room temperature for 30 minutes, followed by dropping 5 ml of a 10% HCl aqueous solution and filtration to obtain 1.3 g of a white solid.
Next, 1.1 g of the thus obtained white solid, 1.0 g of Amberlyst (registered trade name) 15 (commercial name of Rohm & Haas Company), and 20.0 ml of tetrahydrofuran were added to a 50 ml round bottom flask equipped with a condenser to provide a mixture, followed by stirring for reaction at 70°C for 5 hours. After completion of the reaction, the reaction solution was filtered under reduced pressure, after which the solvent was removed from the solution to obtain a yellow solid. This yellow solid was purified by recrystallization (hexane/ethyl acetate = 1/1) to obtain 0.9 g of a white solid. This solid was subjected to measurement of NMR with the results shown below. From the results, it was confirmed that this white solid was made of the polymerizable liquid crystal compound (Z1) (yield: 71%).

$^1$H-NMR(DMSO-d6) δ: 1.2-1.8(m, 8H), 2.60(m, 1H), 3.09(m, 1H), 4.04(m, 2H), 4.55(m, 1H), 5.69(s, 1H), 6.02(s, 1H), 6.99(d, 2H), 7.88(d, 2H), 12.5(s, broad, 1H).

Example 2

Synthesis of polymerizable liquid crystal compound (Z2)

[Chemical Formula 37]

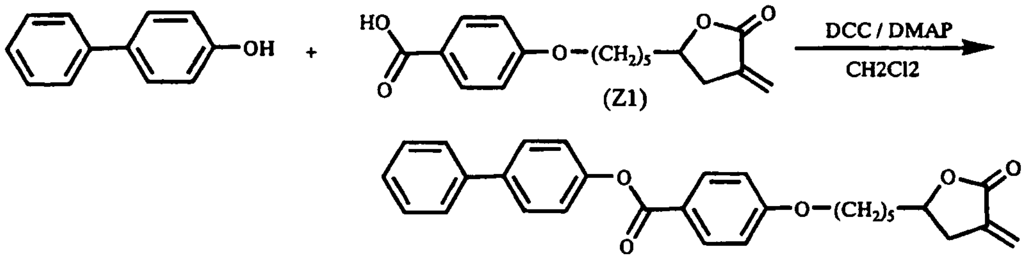


(Z1)

(Z2)

0.6 g (2.0 mmol) of the compound (Z1) obtained in Example 1, 0.3 g (2.0 mmol) of 4-hydroxybiphenyl, 0.008 g of N,N-dimethyl-4-aminopyridine (DMAP) and a small amount of BHT were suspended in 10 ml of methylene chloride under stirring at room temperature, to which a solution of 0.5 g (2.5 mmol) of dicyclohexylcarbodiimide (DCC) dissolved in 5 ml of methylene chloride was added, followed by stirring overnight. The precipitated DCC urea was separated by filtration and the resulting filtrate was successively washed each twice with 50 ml of 0.5N-HCl, 50 ml of a saturated sodium hydrogen carbonate aqueous solution and 50 ml of a saturated saline solution and dried over magnesium sulfate, after which the solvent was distilled off, followed by purification through recrystallization with ethanol to obtain 0.6 g of the intended polymerizable liquid crystal compound (Z2) (yield: 62%).

$^1$H-NMR($CDC1_3$) δ: 1.56(m, 4H), 1.75(m, 2H), 1.85(m, 2H), 2.61(m, 1H), 3.07(m, 1H), 4.06(t, 2H), 4.54(m, 1H), 5.63(d, 1H), 6.24(d, 1H), 6.97(d, 2H), 7.29(d, 2H), 7.35(m, 1H), 7.45(m, 2H), 7.62(m, 2H), 8.17(d, 2H).

It will be noted that the results of observation of the liquid crystal phase of the polymerizable liquid crystal compound (Z2) revealed that upon temperature rise, the compound underwent phase transition into a nematic phase at 121°C and turned into an isotropic liquid state at 122°C. Upon temperature drop, it underwent phase transition into a nematic phase at 122°C and turned into a smectic A phase at 81°C.

Example 3

Synthesis of polymerizable liquid crystal compound (Z3)

[Chemical Formula 38]

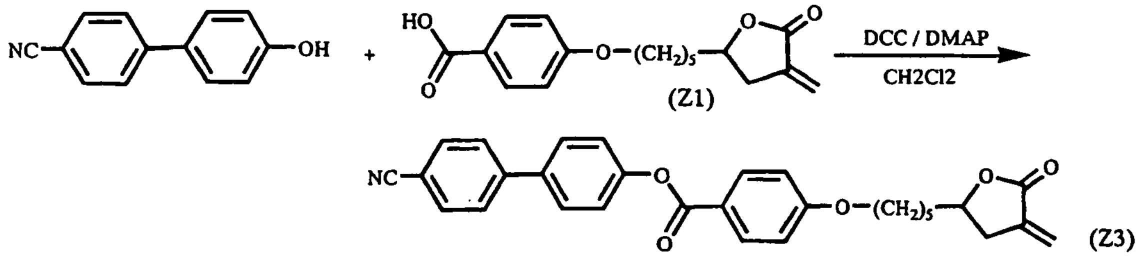


0.6 g (2.0 mmol) of the compound (Z1) obtained in Example 1, 0.4 g (2.0 mmol) of 4-cyano-4'-hydroxybiphenol, 0.008 g of DMAP and a small amount of BHT were suspended in 10 ml of methylene chloride under stirring at room temperature, to which a solution of 0.5 g (2.5 mmol) of DCC dissolved in 5 ml of methylene chloride was added, followed by stirring overnight. The precipitated DCC urea was separated by filtration and the resulting filtrate was successively washed each twice with 50 ml of 0.5N-HCl, 50 ml of a saturated sodium hydrogen carbonate aqueous solution and 50 ml of a saturated saline solution and dried over magnesium sulfate, after which the solvent was distilled off, followed by purification through recrystallization with ethanol to obtain 0.6 g of the intended polymerizable liquid crystal compound (Z3) (yield: 62%).

$^1$H-NMR($CDCl_3$) δ: 1.56(m, 4H), 1.75(m, 2H), 1.85(m, 2H), 2.62(m, 1H), 3.10(m, 1H), 4.05(t, 2H), 4.54(m, 1H), 5.63(d, 1H), 6.24(d, 1H), 6.97(d, 2H), 7.35(d, 2H), 7.71(m, 6H), 8.15(d, 2H).

It will be noted that the results of observation of the polymerizable liquid crystal compound (Z3) revealed that phase transition into a nematic phase occurred at 149°C upon temperature rise.

Example 4

Synthesis of polymerizable liquid crystal compound (Z4)

(1) Synthesis of (Q4)

[Chemical Formula 39]

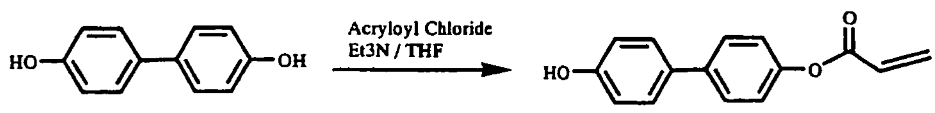


(Q4)

12.7 g of 4,4'-biphenol was dissolved in 50 ml of THF along with 6.86 ml of triethylamine and a small amount of BHT and stirred at room temperature, in which 4.1 ml of acryloyl chloride and 20 ml of THF were dropped over 15 minutes under cooling on a water bath. After the dropping, stirring was continued for 30 minutes and the water bath was removed, followed by stirring overnight while returning to room temperature. The precipitated TEA hydrochloride salt was separated by filtration and about 40 ml of the THF was removed by distillation from the resulting filtrate, to which 50 ml of methylene chloride was further added. The resulting organic phase was successively washed with 50 ml of a 5% HCl aqueous solution, 50 ml of a saturated saline solution, 50 ml of a 10% NaOH aqueous solution and 50 ml of water and dried over magnesium sulfate, followed by distilling off the solvent to obtain a yellow wet solid. This solid was dissolved in 5 ml of ethyl acetate and purified by silica gel column chromatography (column: silica gel 60, 0.063-0.200

mm, made by Merck & Co., eluate: hexane/ethyl acetate = 1/1). The solvent was distilled off from the resulting solution to obtain 2.0 g of a white solid. This solid was subjected to measurement of NMR with the results shown below. From the results, it was confirmed that the white solid was made of the intermediate compound (Q4) (yield: 17%).

$^1$H-NMR(DMSO-d6) δ: 6.15(d, 1H), 6.40(m, 1H), 6.53(d, 1H), 6.87(d, 2H), 7.22(d, 2H), 7.51(d, 2H), 7.61(d, 2H), 9.58(s, 1H).

(2) Synthesis of compound (Z4)

[Chemical Formula 40]

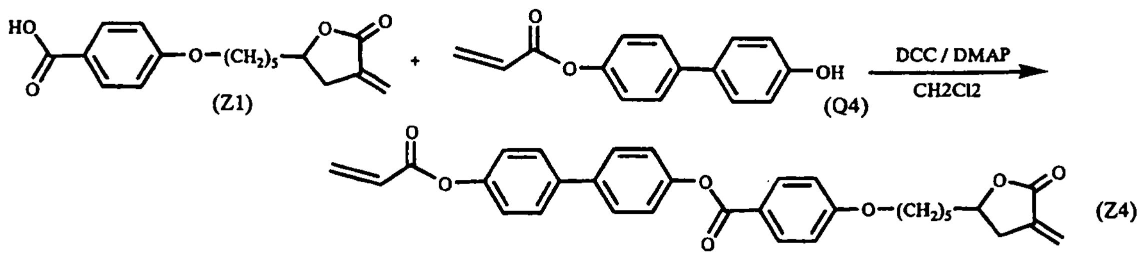


1.5 g of the compound (Z1) obtained in Example 1, 0.8 g of the compound (Q4) obtained above, 0.016 g of DMAP and a small amount of BHT were suspended in 20 ml of methylene chloride under stirring at room temperature, to which a solution of 1.0 g of DCC dissolved in 5 ml of methylene chloride was added, followed by stirring overnight. The precipitated DCC urea was separated by filtration and the resulting filtrate was successively washed each twice with 50 ml of 0.5N-HCl, 50 ml of a saturated sodium hydrogen carbonate aqueous solution and 50 ml of a saturated saline solution and dried over magnesium sulfate, after which the solvent was distilled off, followed by purification through recrystallization with ethanol to obtain 0.45 g of the intended polymerizable liquid crystal compound (Z4).

$^1$H-NMR($CDCl_3$) δ: 1.57(m, 6H), 1.89(m, 2H), 2.61(m, 1H), 3.10(m, 1H), 4.05(m, 2H), 4.56(m, 1H), 5.63(m, 1H), 6.06(d, 1H), 6.24(m, 1H), 6.37(dd, 1H), 6.61(d, 1H), 7.00(d, 2H), 7.27(m, 4H), 7.61(d, 4H), 8.18(d, 2H).

It will be noted that the results of observation of the polymerizable liquid crystal compound (Z4) revealed that upon temperature rise, phase transition into a nematic phase occurred at 139°C and turned into an isotropic liquid state at 193°C.

Example 5

(1) Synthesis of compound (P5)

[Chemical Formula 41]

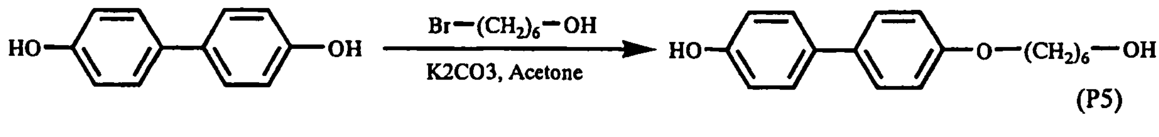


9.5 g (51 mmol) of biphenol, 9.2 g (51 mmol) of 6-bromo-1-hexanol, 13.8 g (100 mmol) of potassium carbonate and 150 ml of acetone were added to a 300 ml bottom round flask equipped with a condenser to provide a mixture,

followed by reaction under stirring at 64°C for 24 hours. After completion of the reaction, the reaction solution was filtered under reduced pressure and the solvent was distilled off under reduced pressure to obtain a yellow wet solid. The solid was recrystallized by use of hexane/ethyl acetate (2/1) to obtain 3.3 g (11.5 mmol) of the intermediate compound (P5) (yield: 23%).

$^1$H-NMR(DMSO-d6) δ: 1.40(m, 6H), 1.72(m, 2H), 3.40(m, 2H), 3.95(m, 2H), 4.34(t, 1H), 6.80(d, 2H), 6.96(d, 2H), 7.46(m, 4H), 9.42(s, 1H).

(2) Synthesis of compound (Q5)

[Chemical Formula 42]

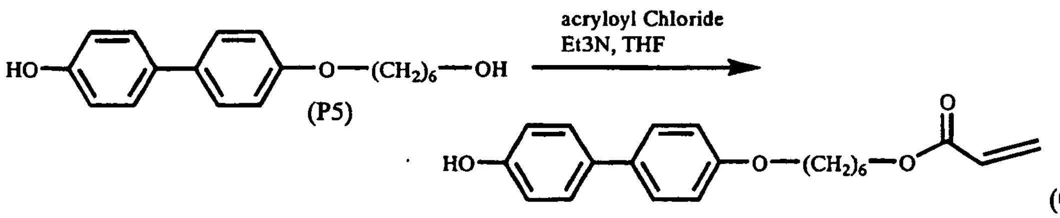


(P5)

(Q5)

2.4 g of the compound (P5) obtained above was dissolved in 10 ml of THF along with 1.15 ml of triethylamine and a small amount of BHT and stirred at room temperature, in which a THF (5 ml) solution of 2.1 ml of acryloyl chloride was dropped over 15 minutes under cooling on a water bath. After the dropping, stirring was continued for 30 minutes and the water bath was removed, followed by stirring overnight while returning to room temperature. The precipitated TEA hydrochloride salt was separated by filtration and about 3/4 of the THF was removed by distillation from the resulting filtrate, to which 50 ml of methylene chloride was further added. The resulting organic phase was successively washed with 50 ml of a saturated sodium hydrogen carbonate aqueous solution, 50 ml of a 0.5N-HCl, and 50 ml of a saturated saline solution and dried over magnesium sulfate, followed by distilling off the solvent to obtain a yellow wet solid. This solid was dissolved in 3 ml of ethyl acetate and purified by silica gel column chromatography (column: silica gel 60, 0.063-0.200 mm, made by Merck & Co., eluate: hexane/ethyl acetate = 1/1). The solvent was distilled off from the resulting solution to obtain 0.9 g of a white solid. This solid was subjected to measurement of NMR with the results shown below. From the results, it was confirmed that the white solid was made of the intermediate compound (Q5) (yield: 33%).

$^1$H-NMR($CDCl_3$) δ: 1.49(m, 4H), 1.72(m. 4H), 3.99(t, 2H), 4.18(m, 2H), 4.86(s, 1H), 5.84(m, 1H), 6.14(m, 1H), 6.37(m, 1H), 6.90(m, 4H), 7.44(m, 4H).

(3) Synthesis of compound (Z5)

[Chemical Formula 43]

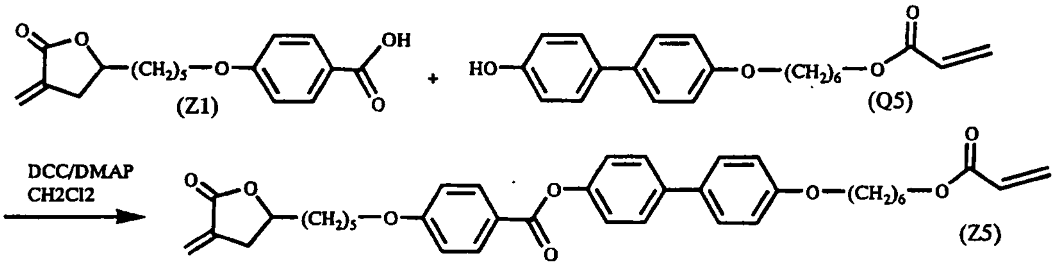


1.5 g of the compound (Z1) obtained in Example 1, 0.8 g of the compound (Q5) obtained above, 0.016 g of DMAP and a small amount of BHT were suspended in 20 ml of methylene chloride under stirring at room temperature, to which a solution of 1.0 g of DCC dissolved in 5 ml of methylene chloride was added, followed by stirring overnight. The precipitated DCC urea was separated by filtration and the resulting filtrate was successively washed each twice with 50 ml of 0.5N-HCl, 50 ml of a saturated sodium hydrogen carbonate aqueous solution and 50 ml of a saturated saline solution and dried over magnesium sulfate, after which the solvent was distilled off, followed by purification through recrystallization with ethanol to obtain 0.45 g of the intended polymerizable liquid crystal compound (Z5).

$^1$H-NMR($CDCl_3$) δ: 1.57(m, 6H), 1.89(m, 2H), 2.61(m, 1H), 3.10(m, 1H), 4.05(m, 2H), 4.56(m, 1H), 5.63(m, 1H), 6.06(d, 1H), 6.24(m, 1H), 6.37(dd, 1H), 6.61(d, 1H), 7.00(d, 2H), 7.27(m, 4H), 7.61(d, 4H), 8.18(d, 2H).

It will be noted that the results of observation of the polymerizable liquid crystal compound (Z5) revealed that upon temperature rise, the compound underwent phase transition into a smectic phase at 89°C and turned into a nematic phase at 150°C.

Example 6

Synthesis of polymerizable liquid crystal compound (Z6)

[Chemical Formula 44]

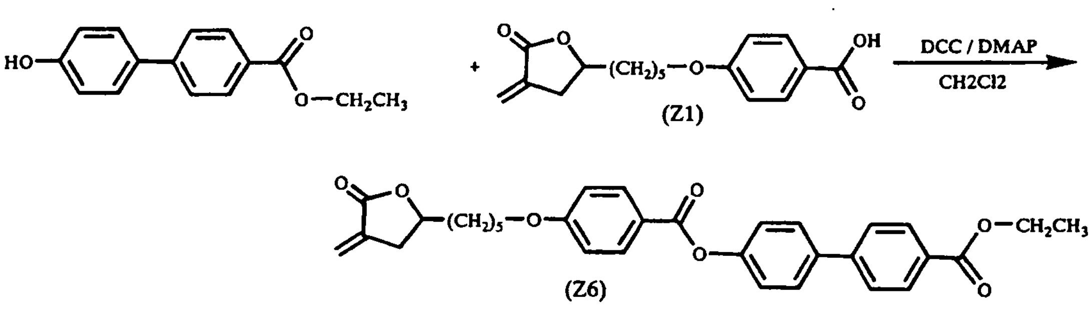


3.0 g (10.0 mmol) of the compound (Z1) obtained in Example 1, 2.4 g (10.0 mmol) of 4'-hydroxy-4-biphenyl carboxylic acid ethyl ester, 0.04 g of DMAP and a small amount of BHT were suspended in 60 ml of methylene chloride under stirring at room temperature, to which a solution of 2.4 g (11.7 mmol) of DCC dissolved in 5 ml of methylene chloride was added, followed by stirring overnight. Thereafter, the precipitated DCC urea was separated by filtration and the resulting filtrate was successively washed each twice with each 70 ml of 0.5N-HCl, a saturated sodium hydrogen carbonate aqueous solution and a saturated saline solution and dried over magnesium sulfate, after which the solvent

was distilled off, followed by purification through recrystallization with ethanol to obtain 3.8 g of the compound (Z6) (yield: 72%).

$^1$H-NMR ($CDCl_3$) δ: 1.40(t, 3H), 1.56(m, 6H), 1.85(m, 2H), 2.60(m, 1H), 3.07(m, 1H), 4.05(t, 2H), 4.42(t, 2H), 4.58 (m, 1H), 5.63(d, 1H), 6.24(d, 1H), 6.98(d, 2H), 7.30(d, 2H), 7.65(m, 4H), 8.19(m, 4H).

The results of observation of a liquid phase of the polymerizable liquid crystal compound (Z6) revealed that upon temperature rise, the compound underwent phase transition into a smectic X phase at 105°C and also into a nematic phase at 177°C.

Example 7

Synthesis of polymerizable liquid crystal compound (Z7)

[Chemical Formula 45]

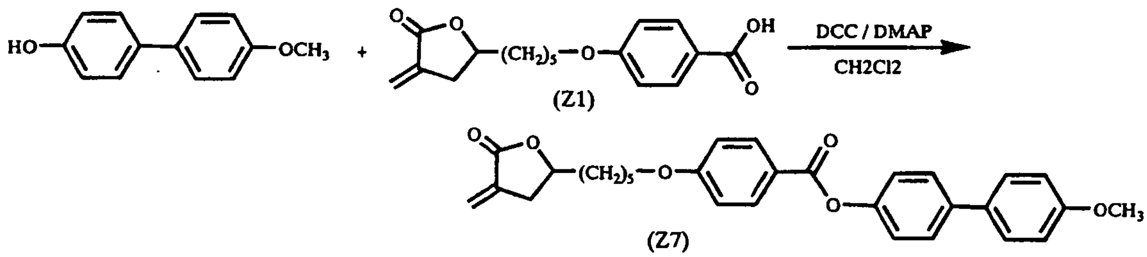


0.6 g (2.0 mmol) of the compound (Z1) obtained in Example 1, 0.4 g (2.0 mmol) of 4-hydroxy-4'-methoxybiphenyl, 0.005 g of DMAP and a small amount of BHT were suspended in 10 ml of methylene chloride under stirring at room temperature, to which 0.5 g (2.5 mmol) of DCC dissolved in 5 ml of methylene chloride was added, followed by stirring overnight. The precipitated DCC urea was separated by filtration and the resulting filtrate was successively washed each twice with each 50 ml of 0.5N-HCl, a saturated sodium hydrogen carbonate aqueous solution and a saturated saline solution and dried over magnesium sulfate, after which the solvent was distilled off, followed by purification through recrystallization operation with ethanol to obtain 0.2 g of the compound (Z7) (yield: 21%).

$^1$H-NMR ($CDCl_3$) δ: 1.55(m, 6H), 1.84(m, 2H), 2.62(m, 1H), 3.06(m, 1H), 3.86(s, 3H), 4.06(t, 2H), 4.55(m, 1H), 5.64(d, 1H), 6.24(d, 1H), 6.97(m, 4H), 7.25(d, 2H), 7.55(m, 4H), 8.18(d, 2H).

The results of observation of a liquid phase of the polymerizable liquid crystal compound (Z7) revealed that upon temperature rise, the compound underwent phase transition into a nematic phase at 114°C and turned into an isotropic liquid state at 176°C.

Example 8

Synthesis of polymerizable compound (Z8)

(1) Synthesis of compound (P8)

[Chemical Formula 46]

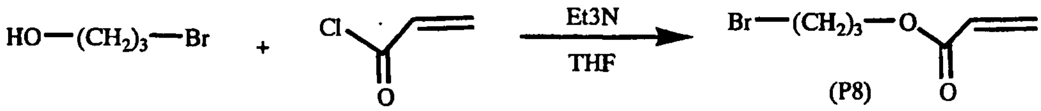


(P8)

19.2 g (138.0 mmol) of 3-bromo-1-propanol was dissolved in 100 ml of THF along with 18.9 ml of triethylamine and a small amount of BHT and stirred at room temperature, in which 12.2 ml (150 mmol) of acryloyl chloride dissolved in 50 ml of THF was dropped over 15 minutes under cooling on a water bath. Thereafter, stirring was continued for 30 minutes and the water bath was removed, followed by stirring overnight while returning to room temperature. The precipitated TEA hydrochloride salt was filtered, and the THF was distilled off from the resulting filtrate, after which 100 ml of diethyl ether was added. The resulting organic phase was successively washed with each 80 ml of a saturated sodium hydrogen aqueous solution, 0.5N-HCl and a saturated saline solution and dried over magnesium sulfate, followed by distilling off the solvent to obtain 18.2 g of the compound (P8).

$^1$H-NMR($CDCl_3$) δ: 2.20(m, 2H), 3.45(t, 2H), 4.33(t, 2H), 5.84(d, 1H), 6.13(m, 1H), 6.44(d, 1H).

(2) Synthesis of compound (Q8)

[Chemical Formula 47]

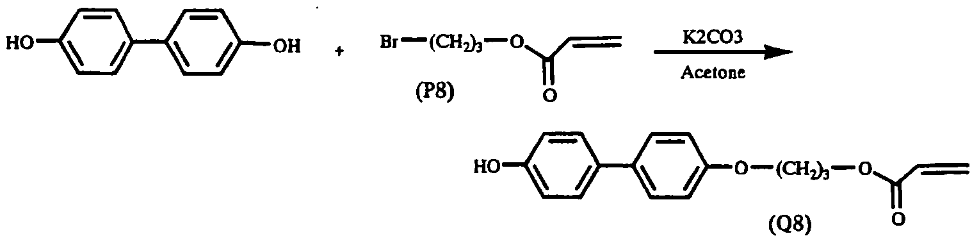


(P8)

(Q8)

17.6 g (94.3 mmol) of 4-hydroxy-4'-biphenol, 18.2 g (94.3 mmol) of the compound (P8) obtained above, 24.0 g (190 mmol) of potassium carbonate and 250 ml of acetone were added to a 500 ml round bottom flask equipped with a condenser to provide a mixture, followed by reaction under stirring at 54°C for 20 hours. After completion of the reaction, the reaction solution was filtered under reduced pressure and the solvent was distilled off under reduced pressure to obtain a yellow wet solid. This solid was purified by silica gel column chromatography (column: silica gel 60, 0.063-0.200 mm, made by Merck & Co., eluate: hexane/ethyl acetate = 2/1). The solvent was distilled off from the resulting solution to obtain 6.1 g of a white solid. This solid was subjected to measurement of NMR with the results shown below. From the results, it was confirmed that the white solid was made of the intermediate compound (Q8) (yield: 22%).

$^1$H-NMR($CDCl_3$) δ: 2.21(m, 2H), 4.13(t, 2H), 4.40(t, 2H), 5.87(d, 1H), 6.15(m, 1H), 6.40(d, 1H), 6.99(d, 2H), 7.52 (d, 2H), 7.68(m, 4H).

(3) Synthesis of polymerizable compound (Z8)

[Chemical Formula 48]

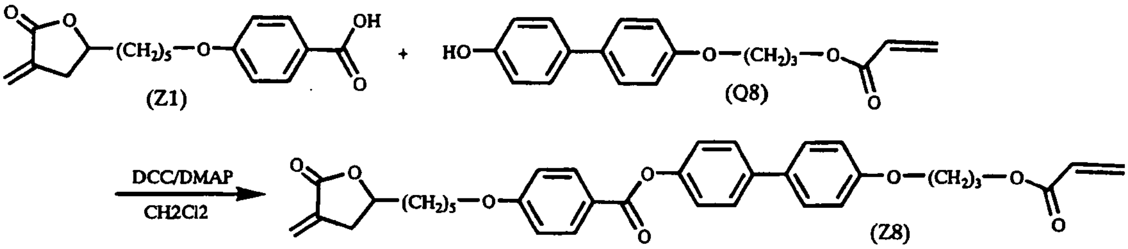


(Z1) (Q8) (Z8)

6.1 g (20.0 mmol) of the compound (Z1) obtained in Example 1, 6.0 g (20.0 mmol) of the compound (Q8) obtained above, 0.08 g of DMAP and a small amount of BHT were suspended in 10 ml of methylene chloride under stirring at room temperature, to which 4.7 g (23.0 mmol) of DCC dissolved in 20 ml of methylene chloride was added, followed by stirring overnight. The precipitated DCC urea was separated by filtration and the resulting filtrate was successively washed each twice with each 60 ml of 0.5N-HCl, a saturated sodium hydrogen carbonate aqueous solution and a saturated saline solution and dried over magnesium sulfate, after which the solvent was distilled off, followed by purification through recrystallization operation with ethanol to obtain 8.8 g of the compound (Z8) (yield: 75%).

$^1$H-NMR($CDCl_3$) δ: 1.53(m, 6H), 1.81(m, 2H), 2.20(m, 2H), 2.60(m, 1H), 3.07(m, 1H), 4.06(t, 2H), 4.12(t, 2H), 4.40 (t, 2H), 4.54(m, 1H), 5.63(d, 1H), 5.85(d, 1H), 6.10(m, 1H), 6.24(d, 1H), 6.42(d, 1H), 6.97(m, 4H), 7.25(m, 2H), 7.54(m, 2H), 7.59(m, 2H), 8.17(d, 2H).

The results of observation of the polymerizable liquid crystal compound (Z8) revealed that upon temperature rise, the compound underwent phase transition into a smectic X phase at 109°C and also into a nematic phase at 144°C and turned into an isotropic liquid state at 168°C.

Example 9

Synthesis of polymerizable compound (Z9)

[Chemical Formula 49]

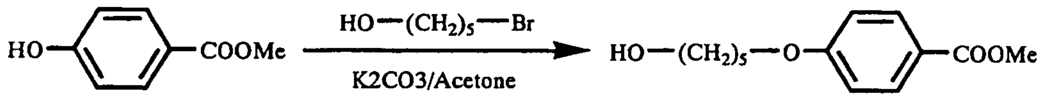


(P9)

5.5 g (36.0 mmol) of methyl 4-hydroxybenzoate, 6.0 g (36.0 mols) of 5-bromo-1-pentanol, 9.0 g (72 mmol) of potassium carbonate and 80 ml of acetonitrile were added to a 200 ml round bottom flask equipped with a condenser to provide a mixture, followed by reaction under stirring at 80°C for 24 hours. After completion of the reaction, the reaction solution was filtered under reduced pressure and the solvent was distilled off under reduced pressure to obtain a yellow wet solid. This solid was purified by silica gel column chromatography (column: silica gel 60, 0.063-0.200 mm, made by Merck & Co., eluate: hexane/ethyl acetate = 1/1). The solvent was distilled off from the resulting solution to obtain 1.8 g of a white solid. This solid was subjected to measurement of NMR with the results shown below. From the results, it was confirmed that the white solid was made of an intermediate compound (P9) (yield: 21%).

$^1$H-NMR($CDCl_3$) δ: 1.5-1.7(m, 4H), 2.85(m, 2H), 3.67(t, 2H), 3.88(s, 3H), 4.02(t, 2H), 6.90(d, 2H), 7.99(d, 2H).

[Chemical Formula 50]

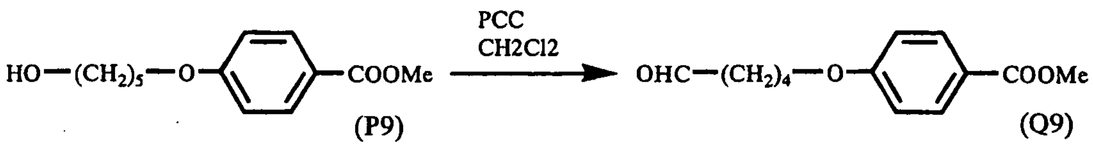


Next, 1.7 g (7.6 mmol) of pyridinium chlorochromate (hereinafter abbreviated as PCC) and 15.0 ml of $CH_2Cl_2$ were placed in a 100 ml three-necked flask equipped with a condenser and mixed under stirring, under which a solution of 1.8 g (7.6 mmol) of the intermediate compound (P9) obtained above dissolved in $CH_2Cl_2$ (15.0 ml) was dropped, followed by further stirring at room temperature for 6 hours. Thereafter, 90 ml of diethyl ether was added to the solution from which an oily matter attached to the wall of the flask was removed, and filtered under reduced pressure, and the solvent was distilled off under reduced pressure to obtain a dark green wet solid.
This solid was purified by silica gel chromatography (column: silica gel 60, 0.063-0.200 mm, made by Merck & Co., eluate: hexane/ethyl acetate = 1/1). The solvent was distilled off from the resulting solution to obtain 1.2 g of a colorless solid. This solid was subjected to measurement of NMR with the results shown below. From the results, it was confirmed that the colorless solid was made of the intermediate compound (Q9) (yield: 67%).

$^1$H-NMR($CDCl_3$) δ: 1.85(m, 4H), 2.54(t, 2H), 3.88(s, 3H), 4.01(t, 2H), 6.91(d, 2H), 7.99(d, 2H), 9.80(s, 1H).

[Chemical Formula 51]

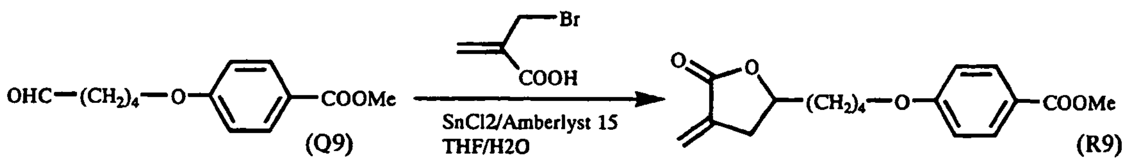


Next, 1.20 g (5.0 mmol) of the intermediate compound (Q9) obtained above, 0.83 g (5.0 mmol) of 2-(bromomethyl)acrylic acid, 0.8 g of Amberlyst (registered trade name) 15 (commercial name of Rohm & Haas Company), 8.0 ml of THF, 0.9 g (5.0 mmol) of tin (II) chloride and 2.0 ml of pure water were added to a 50 ml round bottom flask equipped with a condenser to provide a mixture, followed by reaction under stirring at 70°C for 5 hours. After completion of the reaction, the reaction solution was filtered under reduced pressure and mixed with 40 ml of pure water, to which 50 ml of diethyl ether was added for extraction. The extraction was performed three times.
Anhydrous magnesium sulfate was added for drying to the organic phase obtained after the extraction, and the solvent was distilled off from the resulting solution to obtain 1.3 g of a colorless solid. This solid was subjected to measurement of NMR, with the result that it was confirmed that this white solid was made of the compound (R9) (yield: 85%).

$^1$H-NMR($CDCl_3$) δ: 1.5-1.9(m, 6H), 2.63(m, 1H), 3.06(m, 1H), 3.88(s, 3H), 4.03(t, 2H), 4.54(m, 1H), 5.63(s, 1H), 6.23(s, 1H), 6.90(d, 2H), 7.99(d, 2H).

[Chemical Formula 52]

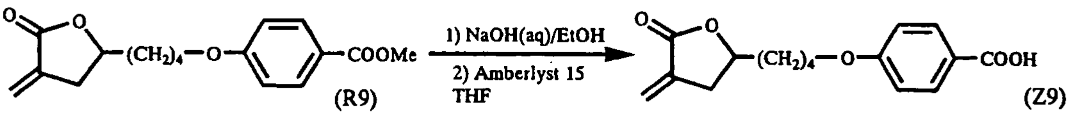


35 ml of ethanol, 1.3 g (4.3 mmol) of the compound (R9) and 5 ml of a 10% sodium hydroxide aqueous solution were added to a 100 ml round bottom flask equipped with a condenser to provide a mixture, followed by reaction under stirring at 85°C for 3 hours. After completion of the reaction, 300 ml of water and the reaction solution were added to a 500 ml beaker and stirred at room temperature for 40 minutes, in which 5 ml of a 10% HCl aqueous solution was dropped, followed by filtration to obtain 1.0 g of a white solid.
Next, 1.0 g of the white solid, 0.9 g of Amberlyst (registered trade name) 15 (commercial name of Rohm & Haas Company), and 20.0 ml of tetrahydrofuran were added to a 50 ml round bottom flask equipped with a condenser to provide a mixture, followed by reaction under stirring at 70°C for 5 hours. After completion of the reaction, the reaction solution was filtered under reduced pressure and the solvent was distilled off from the resulting filtrate to obtain a yellow solid. This yellow solid was purified by recrystallization (hexane/tetrahydrofuran = 2/1) to obtain 0.9 g of a white solid. This solid was subjected to measurement of NMR with the results shown below. From the results, it was confirmed that this white solid was made of the compound (Z9) (yield: 72%).

$^1$H-NMR(DMSI-d6) δ: 1.4-1.8(m, 6H), 2.60(m, 1H), 3.10(m, 1H), 4.06(m, 2H), 4.58(m, 1H), 5.71(s, 1H), 6.03(s, 1H), 7.00(d, 2H), 7.88(d, 2H).

Example 10

Synthesis of polymerizable liquid crystal compound (Z10)

[Chemical Formula 53]

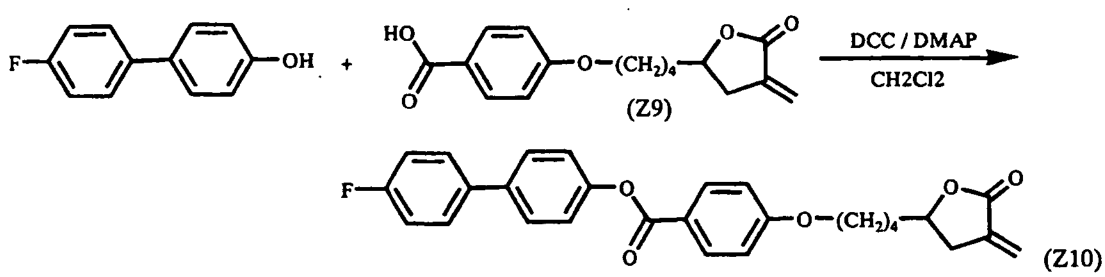


1.0 g (3.4 mmol) of the compound (Z9) obtained in Example 9, 0.7 g (3.4 mmol) of 4-fluoro-4'-hydroxybiphenyl, 0.010 g of DMAP and a small amount of BHT were suspended in 10 ml of methylene chloride under stirring at room temperature, to which 0.8 g (3.8 mmol) of DCC dissolved in 5 ml of methylene chloride was added, followed by stirring overnight. The precipitated DCC urea was separated by filtration and the resulting filtrate was successively washed each twice with each 50 ml of 0.5N-HCl, a saturated sodium hydrogen carbonate aqueous solution and a saturated saline solution and dried over magnesium sulfate, after which the solvent was distilled off, followed by purification through recrystallization operation with ethanol to obtain 0.8 g of the compound (Z10) (yield: 51%).

$^1$H-NMR($CDCl_3$) δ: 1.55-1.90(m, 6H), 2.63(m, 1H), 3.07(m, 1H), 4.06(t, 2H), 4.55(m, 1H), 5.64(d, 1H), 6.24(d, 1H), 6.97(d, 2H), 7.13(m, 2H), 7.26(d, 2H), 7.56(m, 4H), 8.17(d, 2H).

The results of observation of a liquid phase of the polymerizable liquid crystal compound (Z10) revealed that upon temperature rise, the compound underwent phase transition into a smectic X phase at 101°C and into a nematic phase at 117°C, and turned into an isotropic liquid state at 149° C.

Example 11

Synthesis of polymerizable liquid crystal compound (Z11)

[Chemical Formula 54]

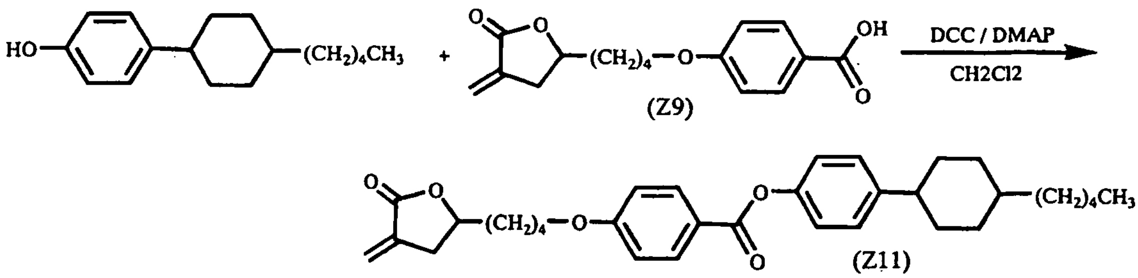


(Z9)

(Z11)

1.0 g (3.4 mmol) of the compound (Z9) obtained in Example 9, 0.9 g (3.4 mmol) of p-(trans-4-pentylcyclohexyl)-phenol, 0.010 g of DMAP and a small amount of BHT were suspended in 10 ml of methylene chloride under stirring at room temperature, to which 0.8 g (3.8 mmol) of DCC dissolved in 5 ml of methylene chloride was added, followed by stirring overnight. The precipitated DCC urea was separated by filtration and the resulting filtrate was successively washed each twice with each 50 ml of 0.5N-HCl, a saturated sodium hydrogen carbonate aqueous solution and a saturated saline solution and dried over magnesium sulfate, after which the solvent was distilled off, followed by purification through recrystallization operation with ethanol to obtain 0.5 g of the compound (Z11) (yield: 28%).

$^1$H-NMR($CDCl_3$) δ: 1.87(t, 3H), 1.05(m, 2H), 1.20-1.55(m, 15H), 1.87(m, 6H), 2.48(m, 1H), 2.64(m, 1H), 3.07(m, 1H), 4.05(t, 2H), 4.55(m, 1H), 5.64(d, 1H), 6.24(d, 1H), 6.95(d, 2H), 7.12(d, 2H), 7.26(d, 2H), 8.13(d, 2H).

The results of observation of a liquid phase of the polymerizable liquid crystal compound (Z11) revealed that upon temperature rise, the compound underwent phase transition into a nematic phase at 90°C and turned into an isotropic liquid state at 170°C. Upon temperature drop, the compound underwent phase transition into a nematic phase at 170° C.

Example 12

Synthesis of polymerizable liquid crystal compound (Z12)

[Chemical Formula 55]

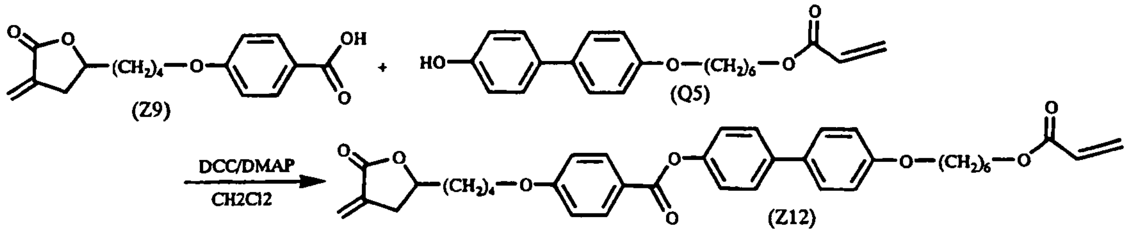


(Z9) (Q5) (Z12)

2.1 g (7.3 mmol) of the compound (Z9) obtained in Example 9, 2.5 g (7.3 mmol) of the compound (Q5) obtained in Example 5, 0.015 g of DMAP and a small amount of BHT were suspended in 30 ml of methylene chloride under stirring at room temperature, to which 1.8 g (9.0 mmol) of DCC dissolved in 5 ml of methylene chloride was added, followed by stirring overnight. The precipitated DCC urea was separated by filtration and the resulting filtrate was washed each twice with each 50 ml of 0.5N-HCl, a saturated sodium hydrogen carbonate aqueous solution and a saturated saline solution and, after drying over magnesium sulfate, the solvent was distilled off, followed by recrystallization operation with ethanol to obtain 1.3 g of the compound (Z12) (yield: 30%).

$^1$H-NMR($CDCl_3$) $\delta$: 1.40-1.90(m, 14H), 2.64(m, 1H), 3.07(m, 1H), 4.00(t, 2H), 4.05(t, 2H), 4.18(t, 2H), 4.54(m, 1H), 5.83(d, 1H), 6.14(m, 1H), 6.25(d, 1H), 6.37(d, 1H), 6.97(d, 2H), 7.26(d, 2H), 7.50(d, 2H), 7.57(d, 2H), 8.17(d, 2H).

The results of observing the liquid crystal phase of the polymerizable liquid crystal compound (Z12) revealed that upon temperature rise, the compound underwent phase transition into a smectic A phase at 90°C and into a nematic phase at 135°C and turned into an isotropic liquid state at 159° C.

Example 13

Synthesis of polymerizable compound (Z13)

[Chemical Formula 56]

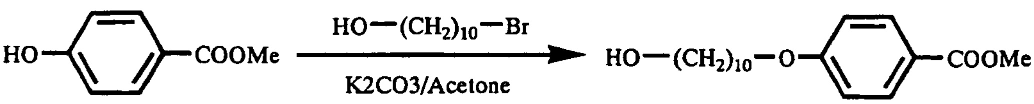


(P13)

16.0 g (105.0 mmol) of methyl 4-hydroxybenzoate, 25.0 g (105.0 mmol) of 10-bromo-1-decanol, 29.0 g (210.0 mmol) of potassium carbonate and 200 ml of acetonitrile were added to a 200 ml round bottom flask equipped with a condenser to provided a mixture, followed by reaction under stirring at 80°C for 48 hours. After completion of the reaction, the reaction solution was filtered under reduced pressure and the solvent was distilled off under reduced pressure to obtain a yellow wet solid. This solid was purified by silica gel chromatography (column: silica gel 60, 0.063-0.200 mm, made by Merck & Co., eluate: hexane/ethyl acetate = 1/1). The solvent was distilled off from the resulting solution to obtain 21.4 g of a white solid. This solid was subjected to measurement of NMR with the results shown below. From the results, it was confirmed that the colorless solid was made of the intermediate compound (P13) (yield: 66%).

$^1$H-NMR($CDCl_3$) $\delta$: 1.2-1.6(m, 14H), 1.8(m, 2H), 3.63(t, 2H), 3.88(s, 3H), 4.02(t, 2H), 6.90(d, 2H), 7.99(d, 2H).

[Chemical Formula 57]

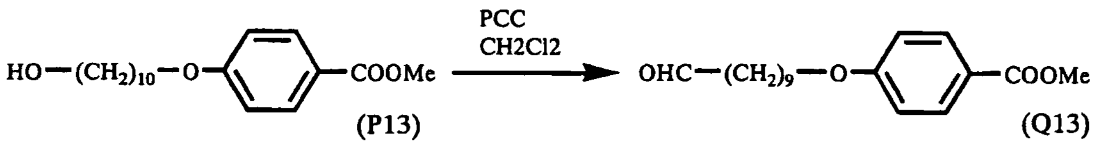


(P13) (Q13)

Next, 15.0 g (69.5 mmol) of pyridinium chlorochromate (hereinafter abbreviated as PCC) and 100.0 ml of $CH_2Cl_2$ were added to a 500 ml three-necked flask equipped with a condenser and mixed under stirring, in which a solution of 21.4 g (69.5 mmol) of the intermediate compound (P13) obtained above dissolved in $CH_2Cl_2$ (100.0 ml) was dropped, followed by further stirring at room temperature for 6 hours. Thereafter, 200 ml of diethyl ether was added to the solution, from which an oily matter attached to the wall of the flask was removed, and subjected to filtration under reduced pressure, followed by distilling off the solvent under reduced pressure to obtain a dark green wet solid.
This solid was purified by silica gel chromatography (column: silica gel 60, 0.063-0.200 mm, made by Merck & Co., eluate: hexane/ethyl acetate = 2/1). The solvent was distilled off from the resulting solution to obtain 16.2 g of a colorless solid. This solid was subjected to measurement of NMR with the results shown below. From the results, it was confirmed that this colorless solid was made of the intermediate compound (Q13) (yield: 76%).

$^1$H-NMR($CDCl_3$) δ: 1.26(m, 10H), 1.62(m, 2H), 1.79(m, 2H), 2.42(m, 2H), 3.88(s, 3H), 4.00(t, 2H), 6.91(d, 2H), 7.99(d, 2H), 9.76(s, 1H).

[Chemical Formula 58]

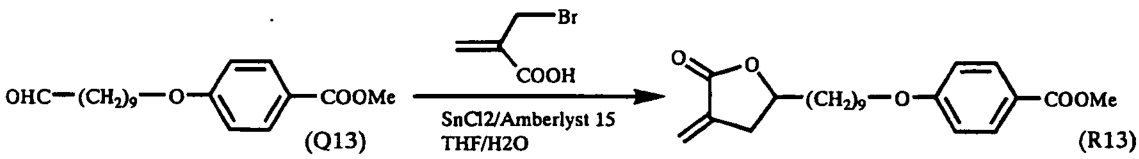


(Q13) (R13)

Next, 16.2 g (53.0 mmol) of the intermediate compound (Q13) obtained above, 8.8 g (53.0 mmol) of 2-(bromomethyl)acrylic acid, 7.4 g of Amberlyst (registered trade name) 15 (commercial name of Rohm & Haas Company), 85.0 ml of THF, 10.1 g (53.0 mmol) of tin (II) chloride and 20.0 ml of pure water were added to a 300 ml round bottom flask equipped with a condenser to provide a mixture, followed by stirring for reaction at 70°C for 20 hours. After completion of the reaction, the reaction solution was filtered under reduced pressure and mixed with 100 ml of pure water, to which 100 ml of diethyl ether was added for extraction. The extraction was performed three times.
Anhydrous magnesium sulfate was added for drying to the organic phase obtained after the extraction and filtered under reduced pressure, and the solvent was distilled off from the resulting solution to obtain 16.4 g of a colorless solid. This solid was subjected to measurement of NMR, from which was confirmed that this colorless solid was made of the intended polymerizable liquid crystal compound (R13) (yield: 83%).

$^1$H-NMR($CDCl_3$) δ: 1.32(m, 12H), 1.60(m, 2H), 1.75(m, 2H), 2.59(m, 1H), 3.02(m, 1H), 3.88(s, 3H), 4.00(t, 2H), 4.50(m, 1H), 5.61(s, 1H), 6.22(s, 1H), 6.90(d, 2H), 7.98(d, 2H).

[Chemical Formula 59]

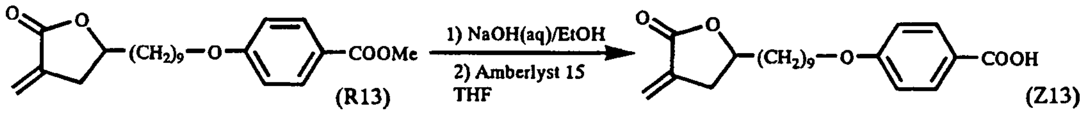


100 ml of ethanol, 16.4 g (43.8 mmol) of the compound (R13) obtained above and 60 ml of a 10% sodium hydroxide aqueous solution were added to a 500 ml round bottom flask equipped with a condenser to provide a mixture, followed by reaction under stirring at 85°C for 5 hours. After completion of the reaction, 1000 ml of water and the reaction solution were added to a 2000 ml beaker and stirred at room temperature for 30 minutes, followed by dropping 60 ml of a 10% HCl aqueous solution and filtration to obtain 14.6 g of a white solid.
Next, 14.6 g of the white solid, 8.0 g of Amberlyst (registered trade name) 15 (commercial name of Rohm & Haas Company), and 100.0 ml of tetrahydrofuran were added to a 300 ml round bottom flask equipped with a condenser to provide a mixture, followed by reaction under stirring at 70°C for 5 hours. After completion of the reaction, the reaction solution was filtered under reduced pressure, after which the solvent was removed from the solution to obtain a yellow solid. This yellow solid was purified by recrystallization (hexane/tetrahydrofuran = 2/1) to obtain 11.6 g of a white solid. This solid was subjected to measurement of NMR with the results shown below. From the results, it was confirmed that this white solid was made of the polymerizable liquid crystal compound (Z13) (yield: 73%).

$^1$H-NMR(DMSO-d6) δ: 1.28(m, 12H), 1.60(m, 2H), 1.72(m, 2H), 2.51(m, 1H), 3.06(m, 1H), 4.04(t, 2H), 4.54(m, 1H), 5.69(s, 1H), 6.02(s, 1H), 7.01(d, 2H), 7.88(d, 2H).

Example 14

Synthesis of polymerizable compound (Z14)

[Chemical Formula 60]

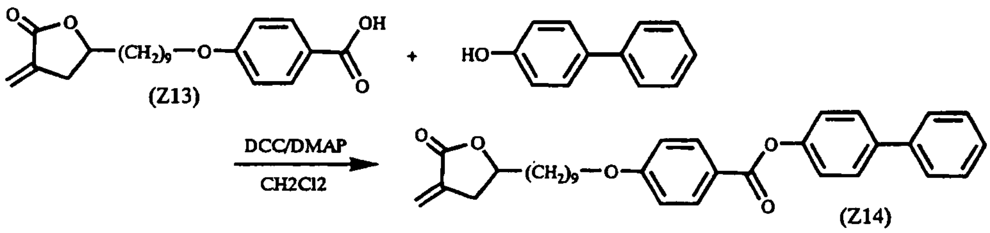


1.0 g (3.0 mmol) of the compound (Z13) obtained in Example 13, 0.5 g (3.0 mmol) of 4-hydroxybiphenyl, 0.010 g of DMAP and a small amount of BHT were suspended in 10 ml of methylene chloride under stirring at room temperature, to which 0.6 g (3.0 mmol) of DCC dissolved in 5 ml of methylene chloride was added and stirred overnight. The precipitated DCC urea was separated by filtration and the resulting filtrate was successively washed each twice with each 50 ml of 0.5N-HCl, a saturated sodium hydrogen carbonate aqueous solution and a saturated saline solution and dried over magnesium sulfate, after which the solvent was distilled off, followed by recrystallization operation with ethanol to obtain 0.3 g of the compound (Z14) (yield: 19%).

$^1$H-NMR ($CDCl_3$) δ: 1.40(m, 14H), 1.80(m, 2H), 2.60(m, 1H), 3.07(m, 1H), 4.06(t, 2H), 4.55(m, 1H), 5.61(d, 1H), 6.24(d, 1H), 6.97(d, 2H), 7.29(d, 2H), 7.35(m, 1H), 7.60(m, 4H), 8.18(d, 2H).

The results of observing the liquid crystal phase of the polymerizable liquid crystal compound (Z14) revealed that upon

temperature rise, the compound underwent phase transition into a smectic X phase at 94°C and into a nematic phase at 103°C and turned into an isotropic liquid state at 115° C.

[2] Polymerizable liquid crystal composition and polymerized product (film)

The compounds used in the following Examples 15 to 22 and Comparative Examples 1 to 5 are those indicated below. The compositions of Examples 15 to 22 and Comparative Examples 1 to 5 are shown in Table 1 (unit is mg).

[Chemical Formula 61]

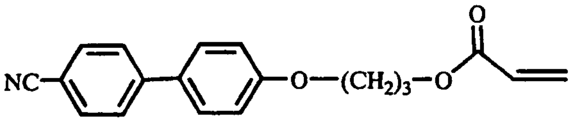

(E1)

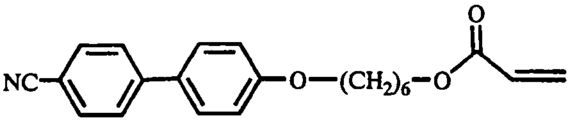

(E2)

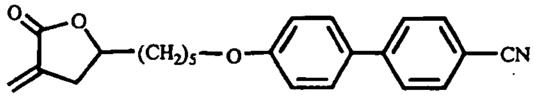

(E3)

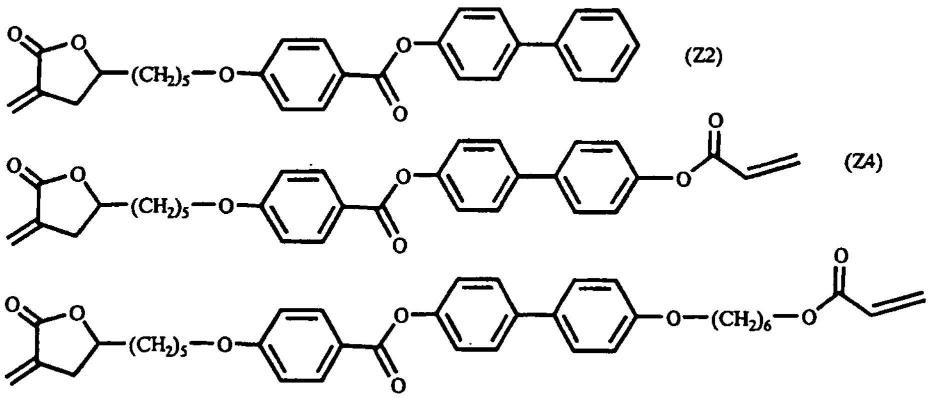

(Z2)

(Z4)

(Z5)

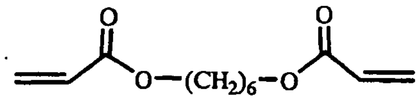

(Aldrich Inc.) (C1)

Table 1

| | Compound E1 | Compound E2 | Compound E3 | Compound Z2 | Compound Z4 | Compound Z5 | Compound C1 |
|---|---|---|---|---|---|---|---|
| Example 15 | 40.0 | 40.0 | - | 20.0 | - | - | - |
| Example 16 | 427 | - | 998 | - | 75.0 | - | - |
| Example 17 | 41.5 | - | 93.5 | - | - | 15.0 | - |
| Example 18 | 36.0 | - | 84.0 | - | - | 30.0 | - |
| Example 19 | 31.5 | - | 73.5 | - | 20.0 | 25.0 | - |
| Example 20 | 27.0 | - | 63.0 | - | 30.0 | 30.0 | - |
| Example 21 | - | - | 37.5 | - | 37.5 | 75.0 | - |
| Example 22 | - | - | - | - | 100.0 | - | - |
| Comparative Example 1 | 50.0 | 50.0 | - | - | - | - | - |
| Comparative Example 2 | 45.0 | - | 105.0 | - | - | - | - |
| Comparative Example 3 | 42.8 | - | 99.7 | - | - | - | 7.5 |
| Comparative Example 4 | 41.6 | - | 93.4 | - | - | - | 15.0 |
| Comparative Example 5 | 36.0 | - | 84.0 | - | - | - | 30.0 |

Example 15

Polymerizable composition and polymerized product (film)

40 mg of polymerizable liquid crystal compound (E1), 40 mg of polymerizable liquid crystal compound (E2), 20 mg of polymerizable liquid crystal compound (Z2), 1.0 mg of Irgacure 369 (commercial name) serving as a photopolymerization initiator and made by Ciba-Geigy K.K., and 0.5 mg of FC4430 (made by Sumitomo 3M, Limited) serving as a surfactant were dissolved in 0.4 ml of cyclohexanone to obtain a polymerizable liquid crystal composition.
This polymerizable liquid crystal composition was coated on a liquid crystal alignment film surface of a liquid crystal alignment film-attached substrate by spin coating (1000 rpm, 20 seconds) and pre-baked on a hot plate of 80°C for 60 seconds, followed by allowing to cool down to room temperature. The polymerizable composition on the substrate was in a liquid crystal state. The liquid crystal alignment film-attached substrate used herein was one wherein a liquid crystal aligning agent (SE-1410, made by Nissan Chemical Industries, Limited) was spin coated onto an ITO surface of an ITO-attached glass substrate, baked at a temperature of 230°C to form a 100 nm thick thin film, followed by rubbing.
Next, the film formed on the liquid crystal alignment film-attached substrate was irradiated with light having an intensity of 2000 $mJ/cm^2$ by use of a metal halide lamp in an atmosphere of nitrogen to polymerize the polymerizable liquid crystal composition. The resulting film had a thickness of 0.8 μm and when observed through a polarizing microscope, it was confirmed that the film was aligned horizontally relative to the substrate surface. The retardation value was found to be at 160 nm with a haze value of 0.2.
When this film was heated on a hot plate of 150°C for 30 minutes, the retardation value was at 148 nm and the haze value was at 2.1.
When this film was immersed in propylene glycol monomethyl ether acetate (PGMEA) at room temperature for 30 minutes, the thickness was at 0.7 μm with a retardation value of 159 nm and a haze value of 0.3.

Comparative Example 1

Polymerizable liquid crystal composition and its polymerized product (film)

50 mg of polymerizable liquid crystal composition (E1), 50 mg of polymerizable liquid crystal compound (E2), 1.0 mg of Irgacure 369 (commercial name) serving as a photopolymerization initiator and made by Ciba-Geigy K.K., and 0.5 mg of FC4430 (made by Sumitomo 3M Limited) serving as a surfactant were dissolved in 0.4 ml of cyclohexanone to obtain a polymerizable liquid crystal composition.
This polymerizable liquid crystal composition was used to obtain a film in the same manner as in Example 15. It will be noted that the composition on the substrate after pre-baking was in a liquid crystal state.
The resulting film had a thickness of 0.7 μm and when observed through a polarizing microscope, it was confirmed that the film was aligned horizontally relative to the substrate surface. The retardation value of the film was at 120 nm and a haze value was at 0.2.
When this film was heated on a hot plate of 150°C for 30 minutes, the retardation value was at 69 nm and the haze value was at 0.8.
When this film was immersed in PGMEA at room temperature for 30 minutes, the thickness was at 0.4 μm with a retardation value of 0 nm and a haze value of 0.8.
The results of Example 15 and Comparative Example 1 are summarized in Tables 2 and 3.
Table 2

| | No heating | | After heating under 150°C/30 minutes | | |
|---|---|---|---|---|---|
| | Δnd (nm) | Haze value | Δnd (nm) | Haze value | Δnd (%) |
| Example 15 | 160 | 0.2 | 148 | 2.1 | 93 |
| Comparative Example 1 | 120 | 0.2 | 69 | 21.0 | 58 |

* Δnd is a retardation value.
* Δnd (%) is a value resulting from the following equation.

$$\Delta nd\ (\%) = \Delta nd\ (\text{after heating under } 150^{\circ}\text{C and 30 minutes}) / \Delta nd\ (\text{no heating}) \times 100$$

Table 3

| | Prior to immersion in PGMEA | | After immersion in PGMEA for 30 minutes | | |
|---|---|---|---|---|---|
| | Δnd (nm) | Haze value | Δnd (nm) | Haze value | Δnd (%) |
| Example 15 | 160 | 0.2 | 159 | 0.3 | 99 |
| Comparative Example 1 | 120 | 0.2 | 0 | 0.8 | 0 |

* Δnd is a retardation value.
* Δnd (%) is a value resulting from the following equation.

$$\Delta nd\ (\%) = \Delta nd\ (\text{after immersion in PGMEA for 30 minutes}) / \Delta nd\ (\text{prior to immersion in PGMEA}) \times 100$$

Example 16

Polymerizable liquid crystal composition and its polymerized product (film)

427 mg of polymerizable liquid crystal compound (E1), 998 mg of polymerizable liquid crystal compound (E3), 75 mg of polymerizable liquid crystal compound (Z4), 15.0 mg of Irgacure 369 (commercial name) serving as a photopolymerization initiator and made by Ciba-Geigy K.K., and 5.0 mg of R-30 (made by DIC corporation) serving as a surfactant were dissolved in 3.5 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 1.6 μm and when observed through a polarizing microscope, it was confirmed that the film was aligned horizontally relative to the substrate surface. The retardation value was found to be at 300 nm with a haze value of 0.1.
When this film was heated on a hot plate of 200°C for 1 hour, the retardation value was at 290 nm and the haze value was at 0.1. Upon heating on a hot plate of 230°C for 1 hour, the retardation value was at 265 nm and the haze value was at 0.1.

Example 17

Polymerizable liquid crystal composition and its polymerized product (film)

41.5 mg of polymerizable liquid crystal compound (E1), 93.5 mg of polymerizable liquid crystal compound (E3), 15.0 mg of polymerizable liquid crystal compound (Z5), 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 1.6 μm and when observed through a polarizing microscope, it was confirmed that the film was aligned horizontally relative to the substrate surface. The retardation value was found to be at 268 nm with a haze value of 0.1.
When this film was heated on a hot plate of 200°C for 1 hour, the retardation value was at 241 nm and the haze value was at 0.2. After heating on a hot plate of 230°C for 1 hour, the retardation value was at 209 nm and the haze value was at 0.2.

Example 18

Polymerizable liquid crystal composition and its polymerized product (film)

36.0 mg of polymerizable liquid crystal compound (E1), 84.0 mg of polymerizable liquid crystal compound (E3), 30.0 mg of polymerizable liquid crystal compound (Z5), 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 1.6 μm and when observed through a polarizing microscope, it was confirmed that the film was aligned horizontally relative to the substrate surface. The retardation value was found to be at 290 nm with a haze value of 0.1.
When this film was heated on a hot plate of 200°C for 1 hour, the retardation value was at 265 nm and the haze value was at 0.2. After heating on a hot plate of 230° C for 1 hour, the retardation value was at 232 nm and the haze value was at 0.2. The incident angle dependence of the retardation value upon heating is shown in Fig. 1.
When this film was immersed in NMP at room temperature for 30 minutes, the film thickness was at 1.5 μm, the retardation value was at 159 nm, and the haze value was at 0.1.

Example 19

Polymerizable liquid crystal composition and its polymerized product (film)

31.5 mg of polymerizable liquid crystal compound (E1), 73.5 mg of polymerizable liquid crystal compound (E3), 20.0 mg of polymerizable liquid crystal compound (Z4), 25.0 mg of polymerizable liquid crystal compound (Z5), 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 1.6 μm and when observed through a polarizing microscope, it was confirmed that the film was aligned horizontally relative to the substrate surface. The retardation value was found to be at 301 nm with a haze value of 0.1.
When this film was heated on a hot plate of 200°C for 1 hour, the retardation value was at 277 nm and the haze value was at 0.2. After heating on a hot plate of 230°C for 1 hour, the retardation value was at 241 nm and the haze value was at 0.2.
When this film was immersed in NMP at room temperature for 30 minutes, the film thickness was at 1.4 μm, the retardation value was at 203 nm, and the haze value was at 0.2.

Example 20

Polymerizable liquid crystal composition and its polymerized product (film)

27.0 mg of polymerizable liquid crystal compound (E1), 63.0 mg of polymerizable liquid crystal compound (E3), 30.0 mg of polymerizable liquid crystal compound (Z4), 30.0 mg of polymerizable liquid crystal compound (Z5), 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 1.7 μm and when observed through a polarizing microscope, it was confirmed that the film was aligned horizontally relative to the substrate surface. The retardation value was found to be at 299 nm with a haze value of 0.1.
When this film was heated on a hot plate of 200°C for 1 hour, the retardation value was at 262 nm and the haze value was at 0.2. After heating on a hot plate of 230°C for 1 hour, the retardation value was at 234 nm and the haze value was at 0.1.
When this film was immersed in NMP at room temperature for 30 minutes, the film thickness was at 1.4 μm, the retardation value was at 211 nm, and the haze value was at 0.2.

Example 21

Polymerizable liquid crystal composition and its polymerized product (film)

37.5 mg of polymerizable liquid crystal compound (E3), 37.5 mg of polymerizable liquid crystal compound (Z4),

75.0 mg of polymerizable liquid crystal compound (Z5), 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.60 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 0.8 μm and when observed through a polarizing microscope, it was confirmed that the film was aligned vertically relative to the substrate surface. The retardation value was found to be at 37 nm (incident angle: 50°) with a haze value of 3.0.
When this film was heated on a hot plate of 200°C for 1 hour, the retardation value was at 36 nm (incident angle: 50°) and the haze value was at 10.0. After heating on a hot plate of 230°C for 1 hour, the retardation value was at 33 nm (incident angle: 50°) and the haze value was at 11.0.
When this film was immersed in NMP at room temperature for 30 minutes, the film thickness was at 0.7 μm, the retardation value was at 36 nm (incident angle: 50°), and the haze value was at 1.5.

Example 22

Polymerizable liquid crystal composition and its polymerized product (film)

100.0 mg of polymerizable liquid crystal compound (Z4), 1.0 mg of Irgacure 369 (commercial name) serving as a photopolymerization initiator and made by Ciba-Geigy K.K., and 0.2 mg of R-30 (made by DIC corporation) serving as a surfactant were dissolved in 1.40 g of cyclohexanone to obtain a polymerizable liquid crystal composition.
This polymerizable liquid crystal composition was used to form a film in the same manner as in Example 15. In this regard, however, the conditions of spin coating the polymerizable liquid crystal composition on the liquid crystal alignment film surface of a liquid crystal alignment film-attached substrate were those of 200 rpm and 20 seconds and the pre-baking conditions were those conditions of 15 seconds on a hot plate of 80°C. It will be noted that the composition on the substrate after the pre-baking was in a liquid crystal state.
The resulting film had a thickness of 0.7 μm and when observed through a polarizing microscope, it was confirmed that the film was aligned vertically relative to the substrate surface. The retardation value was found to be at 36 nm (incident angle: 50°) with a haze value of 2.7.
When this film was heated on a hot plate of 200°C for 1 hour, the retardation value was at 33 nm (incident angle: 50°) and the haze value was at 5.0. After heating on a hot plate of 230°C for 1 hour, the retardation value was at 27 nm (incident angle: 50°) and the haze value was at 4.3.
When this film was immersed in NMP at room temperature for 30 minutes, the film thickness was at 0.7 μm, the retardation value was at 32 nm (incident angle: 50°), and the haze value was at 4.0.

Comparative Example 2

Polymerizable liquid crystal composition and its polymerized product (film)

45.0 mg of polymerizable liquid crystal compound (E1), 105.0 mg of polymerizable liquid crystal compound (E3), 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition.
This polymerizable liquid crystal composition was used to form a film in the same manner as in Example 15. It will be noted that the composition on the substrate after pre-baking was in a liquid crystal state.
The resulting film had a thickness of 1.6 μm and when observed through a polarizing microscope, it was confirmed that the film was aligned horizontally relative to the substrate surface. The retardation value was found to be at 259 nm with a haze value of 0.1.
When the film was heated on a hot plate of 200°C for 1 hour, the retardation value was at 0 nm and the haze value was at 11.7.
When this film was immersed in NMP at room temperature for 30 minutes, the film thickness was found to be 0 μm.

Comparative Example 3

Polymerizable liquid crystal composition and its polymerized product (film)

42.8 mg of polymerizable liquid crystal compound (E1), 99.7 mg of polymerizable liquid crystal compound (E3), 7.5 mg of polymerizable compound (C1) showing no liquid crystallinity, 1.5 mg of Irgacure 369 (commercial name)

serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition. This polymerizable liquid crystal composition was used to form a film in the same manner as in Example 15. It will be noted that the composition on the substrate after pre-baking was in a liquid crystal state.
The resulting film had a thickness of 1.3 μm and when observed through a polarizing microscope, it was confirmed that the film was aligned horizontally relative to the substrate surface. The retardation value was found to be at 254 nm with a haze value of 0.1.
When this film was heated on a hot plate of 200°C for 1 hour, the retardation value was found to be at 179 nm and the haze value was at 0.5.
When this film was immersed in NMP at room temperature for 30 minutes, the film thickness was at 0.8 μm, the retardation value was at 0 nm and the haze value was at 0.9.

Comparative Example 4

Polymerizable liquid crystal composition and its polymerized product (film)

41.6 mg of polymerizable liquid crystal compound (E1), 93.4 mg of polymerizable liquid crystal compound (E3), 15.0 mg of polymerizable compound (C1) showing no liquid crystallinity, 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition. This polymerizable liquid crystal composition was used to form a film in the same manner as in Example 15. It will be noted that the composition on the substrate after pre-baking was in a liquid crystal state.
The resulting film had a thickness of 1.6 μm and when observed through a polarizing microscope, it was confirmed that the film was aligned horizontally relative to the substrate surface. The retardation value was found to be at 222 nm with a haze value of 0.1.
When this film was heated on a hot plate of 200°C for 1 hour, the retardation value was found to be at 159 nm and the haze value was at 0.1.
When this film was immersed in NMP at room temperature for 30 minutes, the film thickness was at 1.0 μm, the retardation value was at 0 nm and the haze value was at 0.3.

Comparative Example 5

Polymerizable liquid crystal composition and its polymerized product (film)

36.0 mg of polymerizable liquid crystal compound (E1), 84.0 mg of polymerizable liquid crystal compound (E3), 30.0 mg of polymerizable compound (C1) showing no liquid crystallinity, 1.5 mg of Irgacure 369 (commercial name) serving as a photopolymerization initiator and made by Ciba-Geigy K.K., and 0.3 mg of R-30 (made by DIC corporation) serving as a surfactant were dissolved in 0.35 g of cyclohexanone to obtain a polymerizable liquid crystal composition. This polymerizable liquid crystal composition was used to form a film in the same manner as in Example 15. It will be noted that the composition on the substrate after pre-baking was in a liquid crystal state.
The resulting film had a thickness of 1.4 μm and when observed through a polarizing microscope, it was confirmed that the film was aligned horizontally relative to the substrate surface. The retardation value was found to be at 181 nm with a haze value of 0.3.
When this film was heated on a hot plate of 200°C for 1 hour, the retardation value was found to be at 77 nm and the haze value was at 0.1. The incident angle dependence of the retardation value upon heating is shown in Fig. 2.
When this film was immersed in NMP at room temperature for 30 minutes, the film thickness was at 1.0 μm, the retardation value was at 0 nm and the haze value was at 0.2.
The results of Examples 16 to 22 and Comparative Examples 2 to 5 are summarized in Tables 4 and 5. It is to be noted that the tests in Table 5 were carried out after exposing a film to light and baking at 200°C for 1 hour provided that with the case of Comparative Example 2, the retardation value after the baking was found to be at 0, for which a film, not baked, was used.
Table 4

| | No heating | | After heating at 200°C for 1 hour | | | After heating at 230°C for 1 hour | | |
|---|---|---|---|---|---|---|---|---|
| | Δnd (nm) | Haze value | Δnd (nm) | Haze value | Δnd (%) | Δnd (nm) | Haze value | Δnd (%) |
| Example 16 | 300 | 0.1 | 290 | 0.1 | 97 | 265 | 0.1 | 88 |
| Example 17 | 268 | 0.1 | 241 | 0.2 | 90 | 209 | 0.2 | 78 |
| Example 18 | 290 | 0.1 | 265 | 0.2 | 91 | 232 | 0.2 | 80 |
| Example 19 | 301 | 0.1 | 277 | 0.2 | 92 | 241 | 0.2 | 80 |
| Example 20 | 299 | 0.1 | 262 | 0.2 | 88 | 234 | 0.1 | 78 |
| Example 21 | 37 | 3.0 | 36 | 10.0 | 97 | 33 | 11.0 | 89 |
| Example 22 | 36 | 2.7 | 33 | 5.0 | 92 | 27 | 4.3 | 75 |
| Comparative Example 2 | 259 | 0.1 | 0 | 11.7 | 0 | | | |
| Comparative Example 3 | 254 | 0.1 | 179 | 0.5 | 71 | | | |
| Comparative Example 4 | 222 | 0.1 | 159 | 0.1 | 72 | | | |
| Comparative Example 5 | 181 | 0.3 | 77 | 0.1 | 43 | | | |

* Examples 21 and 22 deal with vertical alignment, for which the incident angle was 50°.
* Δnd is a retardation value.
* Δnd (%) is a value resulting from the following equation.

$$\Delta nd\ (\%) = \Delta nd\ (\text{no heating}) / \Delta nd\ (\text{after the respective heating treatments}) \times 100$$

Table 5

| | After heating at 200°C for 1 hour (prior to immersion in NMP) | | After immersion in NMP for 30 minutes | | |
|---|---|---|---|---|---|
| | Δnd (nm) | Haze value | Δnd (nm) | Haze value | Δnd (%) |
| Example 18 | 265 | 0.2 | 159 | 0.1 | 60 |
| Example 19 | 277 | 0.2 | 203 | 0.2 | 73 |
| Example 20 | 262 | 0.2 | 211 | 0.2 | 81 |
| Example 21 | 36 | 10.0 | 36 | 1.5 | 100 |
| Example 22 | 33 | 5.0 | 32 | 4.0 | 97 |
| Comparative Example 2 | 259 | 0.1 | 0 | | 0 |
| Comparative Example 3 | 179 | 0.5 | 0 | 0.9 | 0 |
| Comparative Example 4 | 159 | 0.1 | 0 | 0.3 | 0 |
| Comparative Example 5 | 77 | 0.1 | 0 | 0.2 | 0 |

* In Comparative Example 2, no alignment occurred after heating at 200°C for 1 hour and thus, a non-baked film was used.
* With Examples 21 and 22, the incident angle was set at 50° because of the vertical alignment.
* Δnd is a retardation value.
* Δnd (%) is a value resulting from the following equation.

$$\Delta nd\ (\%) = \Delta nd\ (\text{after immersion in NMP for 30 minutes}) / \Delta nd\ (\text{prior to immersion in NMP}) \times 100$$

The compounds employed in the following examples 23 to 27 and Comparative Example 6 are as just indicated below. The compositions of Examples 23 to 27 and Comparative Example 6 are shown in Table 6 (wherein the unit is mg).

[Chemical Formula 62]

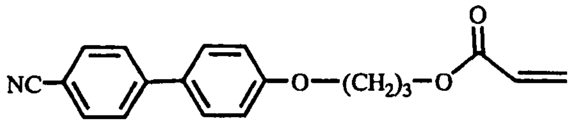

(E1)

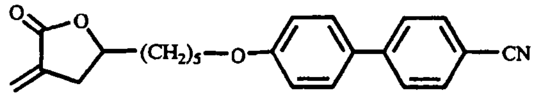

(E3)

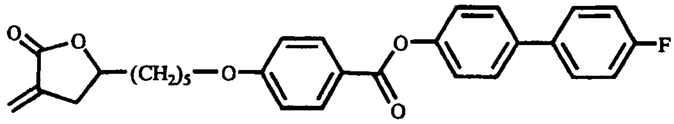

(Z10)

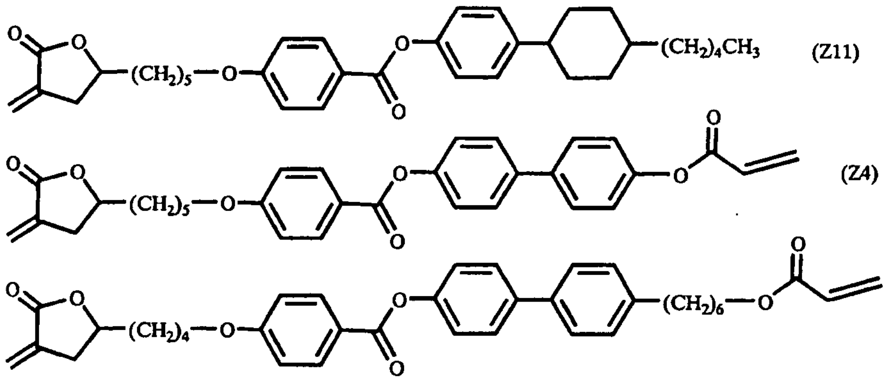

(Z11)

(Z4)

(Z12)

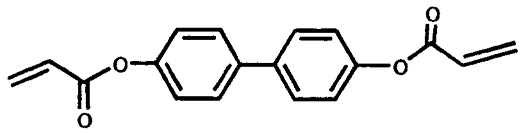

(C2)

Table 6

| | E1 | E3 | Z4 | Z10 | Z11 | Z12 | C2 |
|---|---|---|---|---|---|---|---|
| Example 23 | 81.0 | 54.0 | | | | 15.0 | |
| Example 24 | 72.0 | 48.0 | | | | 30.0 | |
| Example 25 | 61.5 | 51.0 | 7.5 | 15.0 | | 15.0 | |
| Example 26 | 61.5 | 51.0 | 7.5 | | 15.0 | 15.0 | |
| Example 27 | 52.5 | 45.0 | 7.5 | | 30.0 | 15.0 | |
| Comparative Example 6 | 81.0 | 54.0 | | | | | 15.0 |

Example 23

Polymerizable liquid crystal composition and its polymerized product (film)

81.0 mg of polymerizable liquid crystal compound (E1), 54.0 mg of polymerizable liquid crystal compound (E3), 15.0 mg of polymerizable liquid crystal compound (Z12), 1.5 mg of Irgacure 369 (commercial name) serving as a photopolymerization initiator and made by Ciba-Geigy K.K., and 0.1 mg of R-30 (made by DIC corporation) serving as a surfactant were dissolved in 0.35 g of PGMEA to obtain a polymerizable liquid crystal composition.
This polymerizable liquid crystal composition was spin coated (1000 rpm, 20 seconds) on a liquid crystal alignment film

surface of a liquid crystal alignment film-attached substrate and pre-baked on a hot plate of 100°C for 60 seconds, followed by allowing to cool down to room temperature. At this stage, the polymerizable composition on the substrate was in a liquid crystal state. The liquid crystal alignment film-attached substrate used herein was one wherein a liquid crystal aligning agent (SE-1410, made by Nissan Chemical Industries, Limited) was spin coated on an ITO surface of an ITO-attached glass substrate, baked at a temperature of 230°C to form a 100 nm thick thin film, followed by rubbing. The composition on the substrate after the pre-baking was in a liquid crystal state.

Next, the film formed on the liquid crystal alignment film-attached substrate was irradiated with light having an intensity of 2000 mJ/cm$^2$ by use of a metal halide lamp in air to polymerize the polymerizable liquid crystal composition. The resulting film had a thickness of 1.4 μm and when observed through a polarizing microscope, it was confirmed that the film was aligned horizontally relative to the substrate surface. The retardation value was found to be at 255 nm with a haze value of 0.4.
When this film was heated on a hot plate of 150°C for 30 minutes, the retardation value was at 204 nm and the haze value was at 0.2. After heating on a hot plate of 200°C for 1 hour, the retardation value was at 163 nm and the haze value was at 0.1.
When the film was immersed in N-methylpyrrolidone (NMP) for 30 minutes, the film thickness was at 0.9 μm, the retardation value was at 58 nm and the haze value was at 0.5.

Example 24

Polymerizable liquid crystal composition and its polymerized product (film)

72.0 mg of polymerizable liquid crystal compound (E1), 48.0 mg of polymerizable liquid crystal compound (E3), 30.0 mg of polymerizable liquid crystal compound (Z12), 1.5 mg of Irgacure 369 (commercial name) serving as a photopolymerization initiator and made by Ciba-Geigy K.K., and 0.1 mg of R-30 (made by DIC corporation) serving as a surfactant were dissolved in 0.35 g of PGMEA to obtain a polymerizable liquid crystal composition.
This polymerizable liquid crystal composition was used to form a film in the same manner as in Example 23. It will be noted that the composition on the substrate after pre-baking was in a liquid crystal state.
The resulting film had a thickness of 1.4 μm and when observed through a polarizing microscope, it was confirmed that the film was aligned horizontally relative to the substrate surface. The retardation value was found to be at 258 nm with a haze value of 2.8.
When this film was heated on a hot plate of 150°C for 30 minutes, the retardation value was at 210 nm and the haze value was at 0.9. After heating on a hot plate of 200°C for 1 hour, the retardation value was at 178 nm and the haze value was at 0.5.
When this film was immersed in N-methylpyrrolidone (NMP) at room temperature for 30 minutes, the film thickness was at 1.0 μm, the retardation value was at 116 nm, and the haze value was at 0.4.

Example 25

Polymerizable liquid crystal composition and its polymerized product (film)

61.5 mg of polymerizable liquid crystal compound (E1), 51.0 mg of polymerizable liquid crystal compound (E3), 7.5 mg of polymerizable liquid crystal compound (Z4), 15.0 mg of polymerizable liquid crystal compound (Z10), 15.0 mg of polymerizable liquid crystal compound (Z12), 1.5 mg of Irgacure 369 (commercial name) serving as a photopolymerization initiator and made by Ciba-Geigy K.K., and 0.1 mg of R-30 (made by DIC corporation) serving as a surfactant were dissolved in 0.35 g of PGMEA to obtain a polymerizable liquid crystal composition.
This polymerizable liquid crystal composition was used to form a film in the same manner as in Example 23. It will be noted that the composition on the substrate after pre-baking was in a liquid crystal state.
The resulting film had a thickness of 1.4 μm and when observed through a polarizing microscope, it was confirmed that the film was aligned horizontally relative to the substrate surface. The retardation value was found to be at 268 nm with a haze value of 0.3.
When this film was heated on a hot plate of 150°C for 30 minutes, the retardation value was at 215 nm and the haze value was at 0.2. After heating on a hot plate of 200°C for 1 hour, the retardation value was at 170 nm and the haze value was at 0.1.
When this film was immersed in N-methylpyrrolidone (NMP) at room temperature for 30 minutes, the film thickness was at 0.8 μm, the retardation value was at 72 nm, and the haze value was at 0.3.

Example 26

Polymerizable liquid crystal composition and its polymerized product (film)

61.5 mg of polymerizable liquid crystal compound (E1), 51.0 mg of polymerizable liquid crystal compound (E3), 7.5 mg of polymerizable liquid crystal compound (Z4), 15.0 mg of polymerizable liquid crystal compound (Z11), 15.0 mg of polymerizable liquid crystal compound (Z12), 1.5 mg of Irgacure 369 (commercial name) serving as a photopolymerization initiator and made by Ciba-Geigy K.K., and 0.1 mg of R-30 (made by DIC corporation) serving as a surfactant were dissolved in 0.35 g of PGMEA to obtain a polymerizable liquid crystal composition.
This polymerizable liquid crystal composition was used to form a film in the same manner as in Example 23. It will be noted that the composition on the substrate after pre-baking was in a liquid crystal state.
The resulting film had a thickness of 1.4 μm and when observed through a polarizing microscope, it was confirmed that the film was aligned horizontally relative to the substrate surface. The retardation value was found to be at 255 nm with a haze value of 0.1.
When this film was heated on a hot plate of 150°C for 30 minutes, the retardation value was at 211 nm and the haze value was at 0.2. After heating on a hot plate of 200°C for 1 hour, the retardation value was at 180 nm and the haze value was at 0.1.
When this film was immersed in N-methylpyrrolidone (NMP) at room temperature for 30 minutes, the film thickness was at 0.9 μm, the retardation value was at 68 nm, and the haze value was at 0.2.

Example 27

Polymerizable liquid crystal composition and its polymerized product (film)

52.5 mg of polymerizable liquid crystal compound (E1), 45.0 mg of polymerizable liquid crystal compound (E3), 7.5 mg of polymerizable liquid crystal compound (Z4), 30.0 mg of polymerizable liquid crystal compound (Z11), 15.0 mg of polymerizable liquid crystal compound (Z12), 1.5 mg of Irgacure 369 (commercial name) serving as a photopolymerization initiator and made by Ciba-Geigy K.K., and 0.1 mg of R-30 (made by DIC corporation) serving as a surfactant were dissolved in 0.35 g of PGMEA to obtain a polymerizable liquid crystal composition.
This polymerizable liquid crystal composition was used to form a film in the same manner as in Example 23. It will be noted that the composition on the substrate after pre-baking was in a liquid crystal state.
The resulting film had a thickness of 1.4 μm and when observed through a polarizing microscope, it was confirmed that the film was aligned horizontally relative to the substrate surface. The retardation value was found to be at 235 nm with a haze value of 0.6.
When this film was heated on a hot plate of 150°C for 30 minutes, the retardation value was at 192 nm and the haze value was at 0.4. Moreover, after heating on a hot plate of 200°C for 1 hour, the retardation value was at 155 nm and the haze value was at 0.3.
When this film was immersed in N-methylpyrrolidone (NMP) at room temperature for 30 minutes, the film thickness was at 0.8 μm, the retardation value was at 75 nm, and the haze value was at 0.3.

Comparative Example 6

Polymerizable liquid crystal composition and its polymerized product (film)

81.0 mg of polymerizable liquid crystal compound (E1), 54.0 mg of polymerizable liquid crystal compound (E3), 15.0 mg of polymerizable compound (C2), 1.5 mg of Irgacure 369 (commercial name) serving as a photopolymerization initiator and made by Ciba-Geigy K.K., and 0.5 mg of R-30 (made by DIC corporation) serving as a surfactant were dissolved in 0.35 g of PGMEA to obtain a polymerizable liquid crystal composition.
This polymerizable liquid crystal composition was used to form a film in the same manner as in Example 23. It will be noted that the composition on the substrate after pre-baking was in a liquid crystal state.
The resulting film had a thickness of 1.4 μm and when observed through a polarizing microscope, it was confirmed that the film was aligned horizontally relative to the substrate surface. The retardation value was found to be at 231 nm with a haze value of 0.1.
When this film was heated on a hot plate of 150°C for 30 minutes, the retardation value was at 133 nm and the haze value was at 0.1. After heating on a hot plate of 200°C for 1 hour, the retardation value was at 29 nm and the haze value was at 0.1.
When this film was immersed in N-methylpyrrolidone (NMP) at room temperature for 30 minutes, the film thickness was at 0.9 μm, the retardation value was at 5 nm, and the haze value was at 0.4.

The results of Examples 23 to 27 and Comparative Example 6 are summarized in Tables 7 and 8. It will be noted that the tests in Table 8 were carried out after exposing the film to light and baking at 150°C for 30 minutes, followed by further baking at 200°C for 1 hour.

Table 7

| | No heating | | After heating at 150°C for 30 minutes | | | After heating at 150°C for 30 minutes and further heating at 200°C for 1 hour | | |
|---|---|---|---|---|---|---|---|---|
| | Δnd (nm) | Haze value | Δnd (nm) | Haze value | Δnd (%) | Δnd (nm) | Haze value | Δnd (%) |
| Example 23 | 255 | 0.4 | 204 | 0.2 | 80 | 163 | 0.1 | 64 |
| Example 24 | 258 | 2.8 | 210 | 0.9 | 81 | 178 | 0.5 | 69 |
| Example 25 | 268 | 0.3 | 215 | 0.2 | 80 | 170 | 0.1 | 63 |
| Example 26 | 255 | 0.1 | 211 | 0.2 | 83 | 180 | 0.1 | 71 |
| Example 27 | 235 | 0.6 | 192 | 0.4 | 82 | 155 | 0.3 | 66 |
| Comparative Example 6 | 231 | 0.1 | 133 | 0.1 | 58 | 29 | 0.1 | 13 |

* Δnd is a retardation value.
* Δnd (%) is a value resulting from the following equation.

$$\Delta nd\ (\%) = \Delta nd\ (\text{no heating}) / \Delta nd\ (\text{after the respective heating treatments}) \times 100$$

Table 8

| | After heating at 150° C for 30 minutes and further heating at 200°C for 1 hour (prior to immersion in NMP) | | After immersion in NMP for 30 minutes | | |
|---|---|---|---|---|---|
| | Δnd (nm) | Haze value | Δnd (nm) | Haze value | Δnd (%) |
| Example 23 | 163 | 0.1 | 58 | 0.5 | 36 |
| Example 24 | 178 | 0.5 | 116 | 0.4 | 65 |
| Example 25 | 170 | 0.1 | 72 | 0.3 | 42 |
| Example 26 | 180 | 0.1 | 75 | 0.3 | 42 |
| Example 27 | 155 | 0.3 | 68 | 0.2 | 44 |
| Comparative Example 6 | 29 | 0.1 | 5 | 0.4 | 17 |
| * Δnd is a retardation value.<br>* Δnd (%) is a value resulting from the following equation.<br>Δnd (%) = Δnd (immersion in NMP for 30 minutes) / Δnd (prior to immersion in NMP) × 100 | | | | | |

## Claims

1. A polymerizable liquid crystal compound, **characterized by** being represented by the following formula [1]

[Chemical Formula 1]

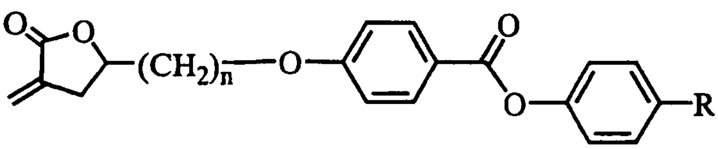

[1]

(wherein R represents an organic group represented by the following formula [A-1], [B-1], [B-2] or [C-1] and n is an integer of 2 to 9)

[Chemical Formula 2]

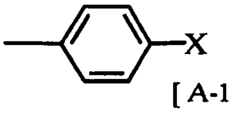

[A-1]

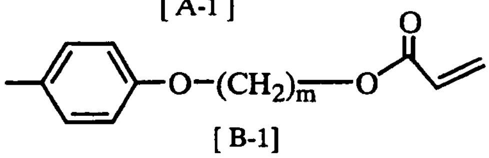

[B-1]

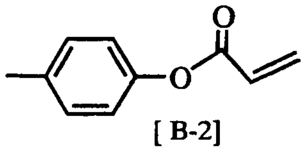

[B-2]

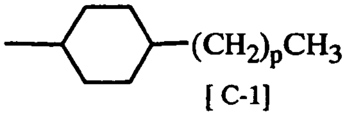

[C-1]

(wherein X represents a hydrogen atom, a halogen atom, a cyano group or an alkoxy group, m is an integer of 2 to 10, and p is an integer of 0 to 6).

2. The polymerizable liquid crystal compound according to claim 1, wherein R is an organic group represented by the formula [B-1] or [B-2].

3. A polymerizable liquid crystal composition comprising at least one polymerizable liquid crystal compound defined in claim 1 or 2.

4. A coating solution for forming an alignment film comprising at least one polymerizable liquid crystal compound defined in claim 1 or 2.

5. An alignment film obtained from the polymerizable liquid crystal composition defined in claim 3.

6. An alignment film obtained from the coating solution for forming an alignment film defined in claim 4.

7. An optical film provided with the alignment film defined in claim 5 or 6.

8. A display device comprising the alignment film defined in claim 5 or 6.

9. A compound represented by the formula [2]

[Chemical Formula 3]

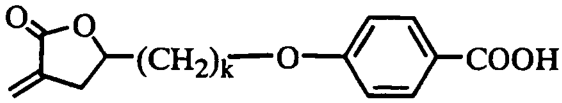

[2]

(wherein k is an integer of 2 to 9).

**Patentansprüche**

1. Polymerisierbare Flüssigkristallverbindung, **dadurch gekennzeichnet, dass** sie durch die folgende Formel [1] dargestellt ist:

[chemische Formel 1]

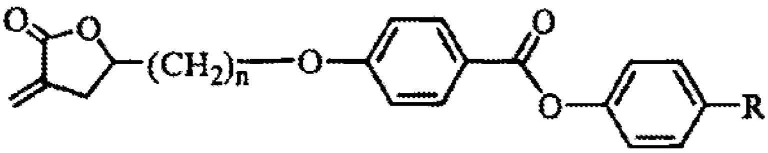

[1]

(worin R für eine organische Gruppe steht, die durch die folgende Formel [A-1], [B-1], [B-2] oder [C-1] dargestellt ist, und n eine ganze Zahl von 2 bis 9 ist)

[chemische Formel 2]

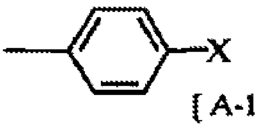

[A-1]

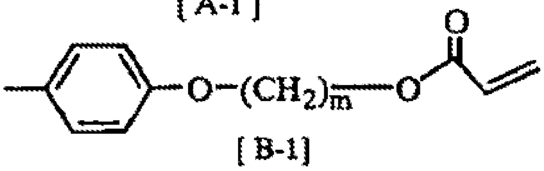

[B-1]

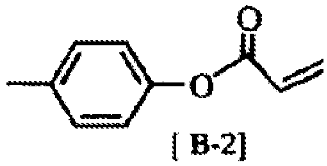

[B-2]

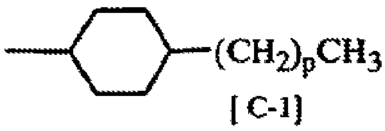

[C-1]

(worin X für ein Wasserstoffatom, ein Halogenatom, eine Cyanogruppe oder eine Alkoxygruppe steht, m eine ganze Zahl von 2 bis 10 ist und p eine ganze Zahl von 0 bis 6 ist).

2. Polymerisierbare Flüssigkristallverbindung nach Anspruch 1, worin R eine organische Gruppe ist, die durch die Formel [B-1] oder [B-2] dargestellt ist.

3. Polymerisierbare Flüssigkristallzusammensetzung, die zumindest eine polymerisierbare Flüssigkristallverbindung nach Anspruch 1 oder 2 umfasst.

4. Beschichtungslösung zur Herstellung eines Ausrichtungsfilms, die zumindest eine polymerisierbare Flüssigkristallverbindung nach Anspruch 1 oder 2 umfasst.

5. Ausrichtungsfilm, der aus einer polymerisierbaren Flüssigkristallzusammensetzung nach Anspruch 3 erhalten worden ist.

6. Ausrichtungsfilm, der aus einer Beschichtungslösung zur Ausbildung eines Ausrichtungsfilms nach Anspruch 4 erhalten worden ist.

7. Optischer Film, der mit einem Ausrichtungsfilm nach Anspruch 5 oder 6 versehen ist.

8. Anzeigevorrichtung, die einen Ausrichtungsfilm nach Anspruch 5 oder 6 umfasst.

9. Verbindung, die durch die Formel [3] dargestellt ist:

[chemische Formel 3]

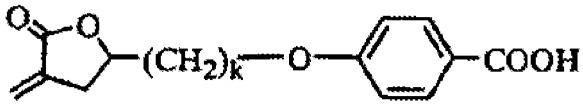

[3]

(worin k eine ganze Zahl von 2 bis 9 ist).

## Revendications

1. Composé cristal liquide polymérisable, **caractérisé en ce qu'**il est représenté par la formule [1] suivante :

[formule chimique 1]

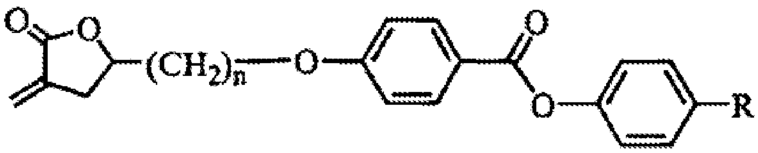

[1]

(dans laquelle R représente un groupe organique représenté par l'une des formules [A-1], [B-1], [B-2] et [C-1] suivantes, et n est un entier de 2 à 9)

[formule chimique 2]

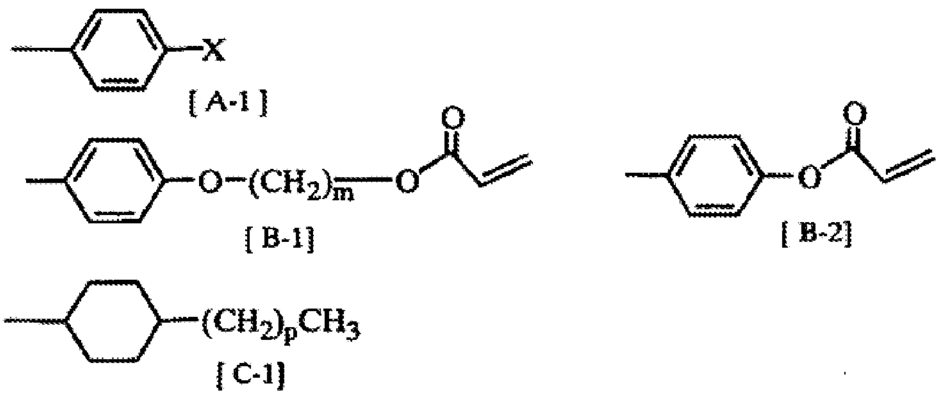

[A-1] [B-1] [B-2] [C-1]

(où X représente un atome d'hydrogène, un atome d'halogène, un groupe cyano ou un groupe alcoxy, m est un entier de 2 à 10, et p est un entier de 0 à 6).

2. Composé cristal liquide polymérisable selon la revendication 1, dans lequel R est un groupe organique représenté par la formule [B-1] ou [B-2].

3. Composition de cristal liquide polymérisable comprenant au moins un composé cristal liquide polymérisable défini dans la revendication 1 ou 2.

4. Solution de revêtement pour former un film d'alignement comprenant au moins un composé cristal liquide polymérisable défini dans la revendication 1 ou 2.

**5.** Film d'alignement obtenu par la composition de cristal liquide polymérisable définie dans la revendication 3.

**6.** Film d'alignement obtenu à partir de la solution de revêtement pour former un film d'alignement définie dans la revendication 4.

**7.** Film optique doté du film d'alignement défini dans la revendication 5 ou 6.

**8.** Dispositif d'affichage comprenant le film d'alignement défini dans la revendication 5 ou 6.

**9.** Composé représenté par la formule [2]

[formule chimique 3]

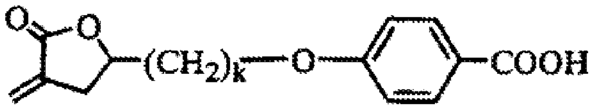

[2]

(dans laquelle k est un entier de 2 à 9).

# FIG.1

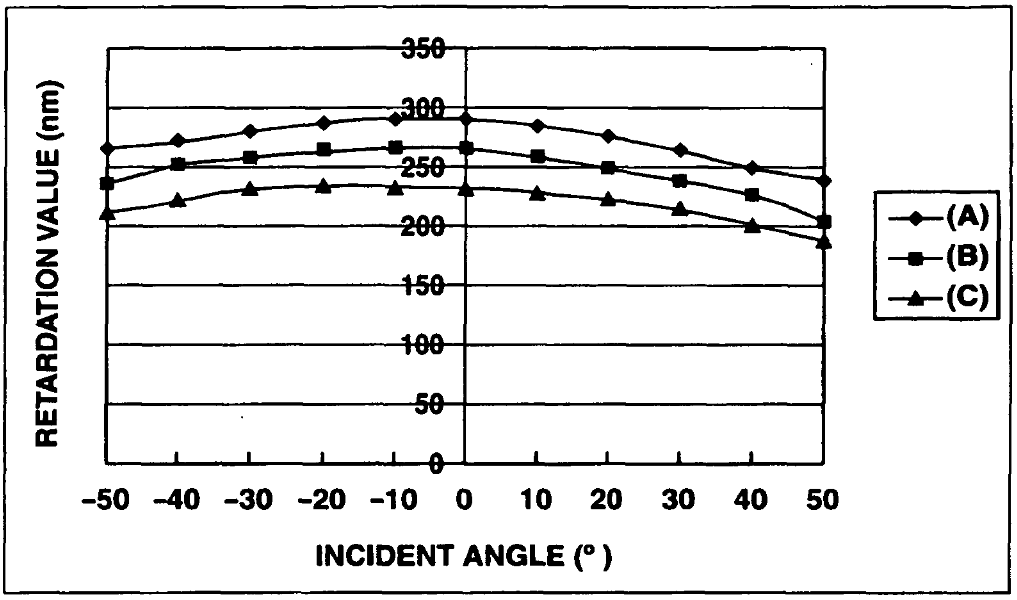

RETARDATION VALUE (nm)
350
300
250
200
150
100
50
0
-50 -40 -30 -20 -10 0 10 20 30 40 50
INCIDENT ANGLE (° )
(A)
(B)
(C)


# FIG.2

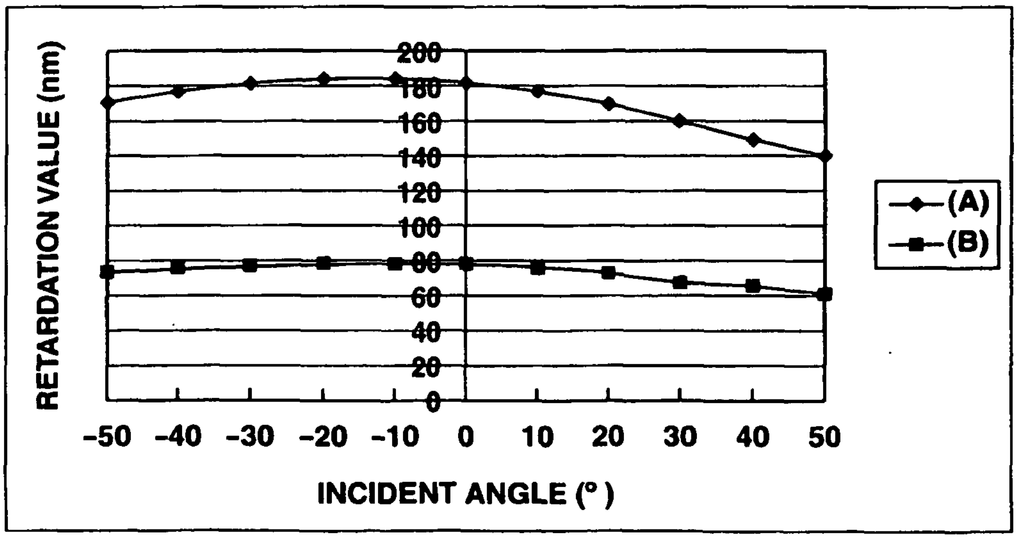

RETARDATION VALUE (nm)
200
180
160
140
120
100
80
60
40
20
0
-50 -40 -30 -20 -10 0 10 20 30 40 50
INCIDENT ANGLE (° )
(A)
(B)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 62070407 A
- JP 9208957 A
- JP 9241249 A
- WO 2006115112 A
- WO 06115033 A
- WO 06115112 A

### Non-patent literature cited in the description

- **P.Talaga ; M.Schaeffer ; C.Benezra ; J.L.Stampf.** *Synthesis,* 1990, 530
- **K.Ramarajan ; K.Kamalingam ; D.J.O'Donnell ; K.D.Berlin.** *Organic Synthesis,* 1983, vol. 61, 56-59